# EUROPEAN PATENT SPECIFICATION

(11) **EP 1 228 229 B1**
(45) Date of publication and mention of the grant of the patent: **09.08.2006**
(21) Application number: 00978569.2
(22) Date of filing: 09.11.2000
(51) Int. Cl.: C12N 15/82, C12N 15/29, C07K 14/415

(54) **CONTROL OF POST-TRANSCRIPTIONAL GENE SILENCING IN PLANTS**
KONTROLLE DER POST-TRANSKRIPTIONELLE GENABSCHALTUNG (GENE SILENCING) IN PFLANZEN
REGULATION DES REPRESSEURS DE GENES POST-TRANSCRIPTIONNELS DE VEGETAUX

(30) Priority: 12.11.1999 US 165199 P; 03.10.2000 US 237432 P
(43) Date of publication of application: 07.08.2002
(73) Proprietor: UNIVERSITY OF SOUTH CAROLINA, Columbia South Carolina 29208 (US)
(72) Inventor: VANCE, Vicki, Columbia, SC 29212 (US)
(74) Representative: Perry, Robert Edward
(86) International application number: PCT/US2000/031109
(87) International publication number: WO 2001/034822

(56) References cited:
- WO-A-01/38512
- BRIGNETI GIANINNA ET AL: "Viral pathogenicity determinants are suppressors of transgene silencing in Nicotiana benthamiana." EMBO (EUROPEAN MOLECULAR BIOLOGY ORGANIZATION) JOURNAL, vol. 17, no. 22, 16 November 1998 (1998-11-16), pages 6739-6746, XP002174427 ISSN: 0261-4189 cited in the application
- ANANDALAKSHMI RADHAMANI ET AL: "A calmodulin-related protein that suppresses posttranscriptional gene silencing in plants." SCIENCE (WASHINGTON D C), vol. 290, no. 5489, 6 October 2000 (2000-10-06), pages 142-144, XP002174428 ISSN: 0036-8075

### SEQUENCE LISTING

<110> Vance, Vicki
<120> Control of Post-Transcriptional Gene Silencing in Plants
<130> AKK-100P
<140> not yet assigned
   <141> 1999-11-12
<160> 2
<170> PatentIn Ver. 2.0
<210> 1
   <211> 948
   <212> DNA
   <213> Nicotiana tabacum
<400> 1
   acaaaagctg agctccaccg cggtgcgccg ctctagaact agtgatcccc cggctgcaga 60
   attcgcacga gaagttttcc ttttagcttc aagttcaacc cttttcacac ttattgtatt 120
   ctacttacat atctcaaatt atcttgaaat taaaggaaaa aaccagtcta agacttacta 180
   gtttagttgt tatacgtgca acatgtgcat ggaatcagtt tctgtaccta gtgttgaaaa 240
   caaatcttat ttctcaagat taaggaagag gttttcactc aaaaaggcaa cgacgactac 300
   aacaacaaca actattacta ctgattatct ttcgatgagt agtagtagca aaagtaataa 360
   tagtggcgag ttagagaggg tatttacata ctttgacgag aatggagatg gaaaagtgtc 420
   accggctgag ctcaggaggt gtgtgaaggc gttaggaggc gaactgacgg tggaggaggc 480
   ggagatggcg gtgaggctat cggattccga cggggatgga ttgttgggtt tggaggattt 540
   tacaaaacta atggaaggaa tggaagagga gagaaataag gagagtgaat tgataggagc 600
   atttggaatg tatgaaatgg aggggagtgg ctacattact cctaagagtt tgaagatgat 660
   gttgagtcga ctcggtgagt caacttccat tgataactgc aaagctatga ttcagagatt 720
   tgatatcaat ggagatggag ttctcaactt tgatgagttc aaagctatga tgacaagtta 780
   actagatttc aacacacaaa tagtgtaatt atacatgtac ataattcttt ggccttgggc 840
   agcttgttgt ttgtttcttg ataaaaagat aaataaattc tgattaatcg ttaaaaaaaa 900
   aaaaaaaaaa aactcgaggg ggttcccgta cccaatctgc cctatagt 948
<210> 2
   <211> 192
   <212> PRT
   <213> Nicotiana tabacum
<400> 2
   Met Cys Met Glu Ser Val Ser Val Pro Ser Val Glu Asn Lys Ser Tyr
    1               5                  10                  15
   Phe Ser Arg Leu Arg Lys Arg Phe Ser Leu Lys Lys Ala Thr Thr Thr
               20                  25                  30
   Thr Thr Thr Thr Thr Ile Thr Thr Asp Tyr Leu Ser Met Ser Ser Ser
           35                  40                  45
   Ser Lys Ser Asn Asn Ser Gly Glu Leu Glu Arg Val Phe Thr Tyr Phe
       50                  55                  60
   Asp Glu Asn Gly Asp Gly Lys Val Ser Pro Ala Glu Leu Arg Arg Cys
   65                  70                  75                  80
   Val Lys Ala Val Gly Gly Glu Leu Thr Val Glu Glu Ala Glu Met Ala
                   85                  90                  95
   Val Arg Leu Ser Asp Ser Asp Gly Asp Gly Leu Leu Gly Leu Glu Asp
               100                 105                 110
   Phe Thr Lys Leu Met Glu Gly Met Glu Glu Glu Arg Asn Lys Glu Ser
           115                 120                 125
   Glu Leu Ile Gly Ala Phe Gly Met Tyr Glu Met Glu Gly Ser Gly Tyr
       130                 135                 140
   Ile Thr Pro Lys Ser Leu Lys Met Met Leu Ser Arg Leu Gly Glu Ser
   145                 150                 155                 160
   Thr Ser Ile Asp Asn Cys Lys Ala Met Ile Gln Arg Phe Asp Ile Asn
                   165                 170                 175
   Gly Asp Gly Val Leu Asn Phe Asp Glu Phe Lys Ala Met Met Thr Ser
               180                 185                 190

## Description

### Background of the Invention

Post-transcriptional gene silencing (PTGS) is an ancient eukaryotic regulatory mechanism (Bingham, 1997) in which a particular RNA sequence is targeted and destroyed. The process may be triggered when multiple copies of a transgene (or a transgene and a homologous endogenous gene) are present in the same genome (for recent reviews of PTGS see Depicker and Van Montagu, 1997; Grant, 1999). Double stranded RNA induces PTGS in many systems (Metzlaff *et al*., 1997; Montgomery and Fire, 1998; Waterhouse *et al.,* 1999) and, in plants, it can also be triggered by cytoplasmically replicating viruses many of which produce double stranded RNA replication intermediates (Baulcombe, 1999; Kumagai *et al*., 1995; Ruiz *et al*., 1998). Once the mechanism has been triggered, any homologous RNA is degraded whether it is transcribed from the transgene, the endogenous gene or from the viral RNA. The fact that plant viruses can act both as inducers and as targets of post-transcriptional gene silencing (AJ-Kaff *et al*., 1998; Matzke and Matzke, 1995; Ratcliff *et al*., 1997) has led to the idea that PTGS may have evolved as an antiviral defense mechanism in plants. The related processes in other eukaryotic organisms may serve a similar function.

If PTGS is an antiviral defense mechanism in plants, then it is not surprising that some plant viruses have evolved a counter-defense. Our recent work and that of others has identified a plant viral protein, HC-Pro, that interferes with the induction of post-transcriptional gene silencing (Anandalakshmi *et al*., 1998; Brigneti *et al*., 1998; Kasschau and Carrington, 1998). This result further supports the idea that PTGS may be linked to natural antiviral resistance systems in plants. and opens the door to a new approach to understanding gene silencing in plants.

The identification of a viral suppressor of PTGS stems from studies of synergistic viral disease, in which coinfection with two heterologous viruses leads to much more severe symptoms than does infection with either virus alone. Many such synergistic diseases involve a member of the potyvirus group of plant viruses (Vance, 1991; Vance *et al*., 1995). Previously, it was found that transgenic plants expressing the 5' proximal region of the tobacco etch potyviral (TEV) genome (termed the P1/HC-Pro sequence) develop synergistic disease when infected with any of a broad range of plant viruses (Pruss *et al*., 1997). This result suggested that expression of the P1/HC-Pro sequence might interfere with a general antiviral system in plants, thereby permitting viruses to accumulate beyond the normal host mediated limits. The general antiviral system was hypothesized to be post-transcriptional gene silencing (Pruss *et al*., 1997). To test this hypothesis, the effect of P1/HC-Pro expression on post-transcriptional gene silencing was tested in two different silencing systems. The results indicate that Pl/HC-Pro acts as a suppressor of both transgene-induced (Anandalakshmi *et al*., 1998; Kasschau and Carrington, 1998) and virus-induced gene silencing (Anandalakshmi *et al*., 1998; Brigneti *et al*., 1998). Further experiments indicated that the suppression of PTGS is mediated by the HC-Pro protein, that the RNA is not sufficient for suppression and that the P1 protein may act as a nonessential accessory protein in the suppression (Anandalakshmi *et al*., 1998; Brigneti *et al*., 1998).

Expression of P1/HC-Pro in transgenic plants confers a number of phenotypes, some potentially useful, others detrimental. As discussed above, P1/HC-Pro interferes with post-transcriptional gene silencing. This phenotype is potentially useful. PTGS limits the level of expression from transgenes and is a serious problem in many biotechnology applications, and HC-Pro can be used to directly counter PTGS. However, suppression of PTGS is not the only process affected by HC-Pro. The P1/HC-Pro transgenic plants have an altered response to viral plant pathogens (and possibly to other pathogens as well). Our early experiments showed that PI/HC-Pro-expressing transgenics respond to infection with potato virus X or tobacco mosaic virus with a systemic necrosis (Pruss *et al.,* 1997). However, in other cases the response of these plants to a viral pathogen resembles systemic acquired resistance (SAR). Under conditions where the *N* resistance gene of tobacco is active, the plants respond to TMV inoculation with fewer and smaller lesions than wild control plants (the classic SAR response). The same lines are completely resistant to infection with tomato ringspot nepovirus, although the control plants are susceptible. The mechanism of this induced resistance is unknown. Expression of P1/HC-Pro in transgenic plants also confers several unique developmental characteristics. The first leaves of the germinating plants are pointed rather than being rounded (the pointed character is a mature leaf character while rounded is the appropriate immature character). The plants grow slowly with reduced root biomass, and a differentiated tumor develops at the junction of the stem and the root. Finally, the flowers show delayed and reduced pigmentation. This result suggests that PTGS is used by plants as one aspect of developmental gene regulation.

To fully utilize transgenic plants, the mechanisms behind gene expression and suppression need to be controlled. In particular, methods for modulating gene expression in plants are needed.

### Summary of the Invention

Plant proteins have been identified that interact with HC-Pro in the yeast two-hybrid system. One of these proteins, like HC-Pro itself, interferes with both the initiation and the maintenance of PTGS. This plant regulator of silencing is a novel calmodulin-related protein (described herein as "rgs-Cam"), and studies of its function should provide important clues to elucidate other steps in the signalling pathway that regulates PTGS.

One aspect of the invention is a rgs-CaM polypeptide that rgs-CaM polypeptide, wherein said polypeptide comprises:
(a) at least a fragment of the polypeptide of SEQ ID NO:2; or
(b) an amino acid sequence encoded by a nucleotide sequence having at least 70% identity to SEQ ID NO:1.

Another aspect of the invention is an isolated polynucleotide encoding rgs-CaM polypeptide that an isolated polynucleotide encoding a rgs-CaM polypeptide, wherein said polynucleotide comprises:
(a) the nucleotide sequence that encodes at least a fragment of the polypeptide of SEQ ID NO:2; or
(b) a nucleotide sequence having at least 70% identity to SEQ ID NO: 1.

A further aspect of the invention is a method of expressing a foreign gene or an endogenous plant gene that has been introduced into plant cells, plant protoplasts or whole plants, the method comprising supplying to said plant cells, plant protoplasts or whole plants a booster sequence comprising a polynucleotide as defined above.

Some portion of the booster sequence may be expressed either individually or fused to other sequences, a modified version of the booster sequence, a related sequence from another plant, or any portion or modified version of that related sequence sufficient to encode a polypeptide exhibiting rgs-CaM activity, expressed either individuaiiy or fused to another sequence. The booster sequence enhances the expression of foreign genes or endogenous plant genes that are introduced to the plant by employing any known methodology. One example of such known methods includes the expression of introduced genes from one or more copies of a gene stably incorporated into the plant genome. Another example of such known methods is the expression of genes introduced via plant viral expression vectors.

The process of enhancing gene expression may be carried out in various forms. For example, the booster sequence may be provided to the plant in a variety of ways. It may be provided by infection with a modified, transgenic virus that expresses the booster sequence as a viral gene product during its natural life cycle. Alternatively, the booster sequence may be introduced through use of a transgenic host plant expressing the booster sequence as an introduced gene. The booster sequence may also be introduced using the same viral expression vector utilized to express the introduced foreign or endogenous gene of interest. A transient expression system may be employed to temporarily express the booster sequence or a two-component co-infection system may be used wherein two viruses are required for successful boosting of gene expression. In the two-component co-infection system, one virus expresses the booster sequence while the other virus expresses the introduced gene of interest.

In addition, the booster sequence may be used to enhance expression of any introduced gene, including foreign genes or endogenous plant genes. The genes to be boosted may be introduced to the plant in a variety of ways. Foreign or endogenous genes may be introduced by means of stable transformation into the genome of the plant employing any of the known technologies used for this process. Alternatively, the foreign or endogenous gene may be introduced using any plant viral expression vector.

More specifically, the present invention involves a method of expressing a foreign gene or an endogenous plant gene that has been introduced into plant material, which includes plant cells, plant protoplasts, or whole plants, wherein the improvement comprises the supplying of a booster sequence comprising a polynucleotide which encodes at least one polypeptide having rgs-CaM activity to the plant material so that expression of said foreign gene or endogenous plant gene is enhanced. The plant gene may be a foreign gene not naturally occurring in the plant material prior to being introduced therein, or an endogenous plant gene that was naturally occurring in the plant material prior to being introduced as an additional copy or additional copies of the endogenous gene. The booster sequence supplied may comprise the coding region for rgs-CaM, and the booster sequence may be expressed independently or fused to other sequences.

The foreign or endogenous gene may be introduced to the plant via a viral expression vector with the booster sequence being supplied by expression from the same viral vector; introduced via a viral expression vector with the booster sequence being supplied by expression of one or more DNA copies of the booster sequence stably incorporated into the plant's genome: or introduced via a viral expression vector with the booster sequence being expressed from a transient expression system containing one or more DNA copies of said booster sequence. A two-component viral vector system may be utilized with one viral component expressing the booster sequence and the other viral component expressing the introduced gene. The introduced gene may be a foreign gene or endogenous plant gene introduced via a viral expression vector with the booster sequence being supplied by co-infection with a modified or transgenic virus that expresses the booster sequence, or introduced via a viral expression vector having the gene fused to the structural gene of interest of said viral expression vector.

In addition, the foreign gene or endogenous plant gene may be introduced into a plant genome via any mode of stable transformation of one or more DNA copies of the introduced gene, with the booster sequence being supplied prior to, during, or after introduction of the foreign gene or endogenous plant gene via stable transformation procedures so that it enhances either the expression of the introduced gene product or the number or proportion of transformant plants that express the introduced gene product. In this aspect, the booster sequence may be supplied via expression from one or more DNA copies of the booster sequence stably incorporated into the plant genome prior to, during, or after transformation of the plant material with said introduced gene product.

### Brief Description of the Drawings

**Figure 1.** Northern blot analysis showing that the rgs-CaM message is greatly overexpressed in the leaves of rgs-CaM transgenic lines (lane 2) compared to control non-transgenic plants (lane 1).
**Figure 2.** Stem-root junctions showing tumor in P1/HC-Pro transgenic line. (A) Normal tobacco control; (B) cross-sectional view showing cellular organization in normal control; (C) P1/HC-Pro transgenic tobacco; (D) cross-sectional view, showing aberrant organization in tumor.
**Figure 3.** Stem-root junctions showing tumor in rgs-CaM transgenic line. (A) normal *N. benthamiana* control; (B) tumor in rgs-CaM transgenic *N. benthamiana*.
**Figure 4.** rgs-CaM transgenic line is deficient in VIGS. (A) GFP transgene is silenced in GFP-transgenic line when infected with PVX-GFP; (B) GFP transgene is not silenced in lines transgenic for both GFP and rgs-CaM when infected with PVX-GFP.
**Figure 5.** Reversal of PTGS by infection with PVX vector expressing rgs-CaM. (A) Agrosilenced GFP transgenic line infected with PVX control vector remains silenced; (B) Agrosilenced GFP transgenic line infected with PVX-rgs-CaM shows reversal of GFP silencing.

### Brief Description of the Sequences

**SEQ ID NO: 1** is the cDNA sequence for rgs-CaM.
**SEQ ID NO: 2** is the amino acid sequence of the rgs-CaM polypeptide encoded by SEQ ID NO: 1.

### Detailed Disclosure of the Invention

Post-transcriptional gene silencing (PTGS) is a fundamental regulatory mechanism operating in diverse types of organisms. Although PTGS was first identified in plant systems, similar pathways appear to operate in filamentous fungi, nematodes, and a variety of animal systems where it is referred to as RNA interference (Fire, 1999; Sharp, 1999; Grant, 1999; Sharp and Zamore, 2000). Double-stranded RNA (dsRNA) induces PTGS in many systems (Montgomery and Fire, 1998; Wianny and Zernicka-Goetz, 2000; Waterhouse and Graham, 1998) and, in plants, it can also be triggered by cytoplasmically replicating viruses, many of which produce dsRNA replication intermediates (Kumagai *et al.,* 1995; Ratcliff *et al.,* 1997). Once the mechanism is activated, any homologous RNA is degraded, whether it is transcribed from the transgene, the endogenous gene, or the viral RNA. The fact that plant viruses can act both as inducers and as targets of posttranscriptional gene silencing (Kumagai *et al.,* 1995;Baulcombe, 1999) has led to the idea that PTGS evolved as a defense mechanism against viruses in plants. HC-Pro is a viral protein that has been shown to suppress PTGS. We have identified a number of proteins that interact with HC-Pro in the yeast two-hybrid system. One of these HC-Pro-interacting proteins, like HC-Pro itself, interferes with both the initiation and the maintenance of PTGS. The plant regulator of silencing is a novel calmodulin-related protein, termed rgs-CaM. Here we teach methods to exploit rgs-CaM as a tool for manipulating the PTGS pathway for biotechnological applications.

Manipulation of the PTGS pathway is an effective means for modulating expression of a gene of interest, or a "target sequence," in a plant. That is, the expression of the target sequence may be enhanced or, if desired, decreased or its silencing maintained. Generally, enhanced expression of the target sequence is effected. By "enhanced expression'' is intended that expression of the target sequence is increased over expression observed in conventional transgenic lines for heterologous sequences and over endogenous levels of expression for homologous sequences, and particularly as compared to expression in systems of either type where the target sequence is subject to PTGS. An rgs-CaM polynucleotide which retains the ability to cause enhanced expression is sometimes referred to herein as a "booster sequence." Heterologous or exogenous sequences comprise sequences that do not occur in the plant of interest in its native state. Homologous or endogenous sequences are those that are natively present in the plant genome. Generally, expression of the target sequence is increased at least about 25%-50%, preferably about 50%-100%, more preferably about 100%-200% and greater. The methods of the invention provide for a substantial increase in expression, yet without apparent negative effects on the plant.

The invention relates to gene silencing, specifically the suppression of gene silencing in plants. However, in another aspect the invention relates to maintaining gene silencing in certain systems where desired. "Gene silencing" is generally used to refer to suppression of expression of a gene. The degree of reduction may be partial or total reduction in production of the encoded gene product. Therefore, the term should not be taken to require complete "silencing" of expression. Methods for gene silencing are known in the art and include co-suppression and antisense suppression. See, for example, PCT/GB98/00442 and PCT/GB98/02862, herein incorporated by reference.

The method of the invention relates to modulating the expression of a target gene or sequence in plants. The target sequence may be endogenous or exogenous in origin. For exogenous sequences, it is recognized that co-suppression or gene-silencing will generally require multiple copies of the exogenous sequence in the plant cell. Multiple copies may be obtained by multiple insertion events from the same transformation event, subsequent transformation of a plant having the exogenous sequence incorporated in the plant genome, genetic crossing, and the like. Additionally, an exogenous sequence may be contained as part of a viral replicon or plant viral expression vector.

The target sequence comprises any sequence of interest, including genes, regulatory sequences, etc. Genes of interest include those encoding agronomic traits, insect resistance, disease resistance, herbicide resistance, sterility, grain characteristics, and the like. The genes may be involved in metabolism of oil, starch, carbohydrates, nutrients, etc. Genes or traits of interest include, but are not limited to, environmental- or stress-related traits, disease-related traits, and traits affecting agronomic performance. Target sequences also include genes responsible for the synthesis of proteins, peptides, fatty acids, lipids, waxes, oils, starches, sugars, carbohydrates, flavors, odors, toxins, carotenoids, hormones, polymers, flavonoids, storage proteins, phenolic acids, alkaloids, lignins, tannins, celluloses, glycoproteins, glycolipids, etc.

Variants or sequences having substantial identity or homology with the enhancer molecules may be utilized in the practice of the invention. That is, the booster sequence may be modified yet still retain the ability to act as a suppressor of post-transcriptional gene silencing. Generally, the booster will comprise at least about 40%-60%, preferably about 60%-80%, more preferably about 80%-90%, and even more preferably about 90%-95% sequence identity with the native booster sequence.

Sequence relationships between two or more nucleic acids or polynucleotides are generally defined as sequence identity, percentage of sequence identity, and substantial identity. See, for example, "Pedestrian Guide to Analyzing Sequence Data Bases" at www.embl-heidelberg.de/~schneide/paper/springer96/springer.html. In determining sequence identity, a "reference sequence" is used as a basis for sequence comparison. The reference may be a subset or the entirety of a specified sequence. That is, the reference sequence may be a full-length gene sequence or a segment of the gene sequence.

Methods for alignment of sequences for comparison are well known in the art. See, for example, Smith *et al*. (1981) *Adv. Appl. Math.* 2:482; Needleman *et al.* (1970) *J. Mol. Biol*. 48:443; Pearson *et al*. (1988) *Proc. Natl. Acad. Sci.* 85:2444; CLUSTAL in the PC/Gene Program by Intelligenetics, Mountain View, California; GAP, BESTFIT, BLAST, FASTA, and TFASTA in the Wisconsin Genetics Software Package, Genetics Computer Group (GCG), 575 Science Drive, Madison, Wisconsin, USA. Preferred computer alignment methods also include the BLASTP, BLASTN, and BLASTX algorithms. See, Altschul *et al*. (1990) *J. Mol. Biol*. 215:403-410.

"Sequence identity" or "identity" in the context of nucleic acid or polypeptide sequences refers to the nucleic acid bases or residues in the two sequences that are the same when aligned for maximum correspondence over a specified comparison window. "Percentage of sequence identity" refers to the value determined by comparing two optimally aligned sequences over a comparison window, wherein the portion of the polynucleotide sequence in the comparison window may comprise additions or deletions as compared to the reference window for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

Polynucleotide sequences having "substantial identity'' are those sequences having at least about 50%-60% sequence identity, generally at least 70% sequence identity, preferably at least 80%, more preferably at least 90%, and most preferably at least 95%, compared to a reference sequence using one of the alignment programs described above. Preferably sequence identity is determined using the default parameters determined by the program. Substantial identity of amino acid sequence generally means sequence identity of at least 50%, more preferably at least 70%, 80%, 90%, and most preferably at least 95%.

Nucleotide sequences are generally substantially identical if the two molecules hybridize to each other under stringent conditions. Generally, stringent conditions are selected to be about 5°C lower than the thermal melting point for the specific sequence at a defined ionic strength and pH. Nucleic acid molecules that do not hybridize to each other under stringent conditions may still be substantially identical if the polypeptides they encode are substantially identical. This may occur, for example, when a copy of a nucleic acid is created using the maximum codon degeneracy permitted by the genetic code.

As noted, hybridization of sequences may be carried out under stringent conditions. By "stringent conditions" is intended conditions under which a probe will hybridize to its target sequence to a detectably greater degree than to other sequences. Stringent conditions are sequence-dependent and will be different in different circumstances. Typically, stringent conditions will be those in which the salt concentration is less than about 1.5 M Na ion, typically about 0.01 to 1.0 M Na ion concentration (or other salts) at pH 7.0 to 8.3 and the temperature is at least about 30° C for short probes (*e*.*g*., 10 to 50 nucleotides) and at least about 60° C for long probes (*e.g*., greater than 50 nucleotides). Stringent conditions may also be achieved with the addition of destabilizing agents such as formamide. Exemplary stringent conditions include hybridization with a buffer solution of 30 to 35% formamide, 1.0 M NaCl, 1% SDS (sodium dodecyl sulphate) at 37° C, and a wash in 1X to 2X SSC (20X SSC = 3.0 M NaCl/0.3 M trisodium citrate) at 50 to 55° C. It is recognized that the temperature, salt, and wash conditions may be altered to increase or decrease stringency conditions. For the post-hybridization washes, the critical factors are the ionic strength and temperature of the final wash solution. See, Meinkoth and Wahl (1984) *Anal. Biochem*. 138:267-284.

As indicated, fragments and variants of the nucleotide sequences of the invention are encompassed herein. By "fragment" is intended a portion of the nucleotide sequence. Fragments may be generated by a number of methods well known in the art, such as by use of commercially available restriction enzymes, exonucleases such as *Bal*31, or by chemical synthesis. Fragments of the booster sequence wiii generally encode polypeptides which retain one or more of the biological activities of the native protein. Activities can be tested and confirmed by following examples set forth herein. Alternatively, fragments of the polynucleotide sequences may or may not retain biological activity. Such sequences may be useful as hybridization probes, as antisense constructs, or as co-suppression sequences. Thus, fragments of a nucleotide sequence may range from at least about 15-20 nucleotides, about 50 nucleotides, about 100 nucleotides, and up to the full-length nucleotide sequence of the particular polynucleotide of interest.

By "variants" is intended substantially similar sequences. For example, for nucleotide sequences, conservative variants include those sequences that, because of the degeneracy of the genetic code, encode the amino acid sequence of the booster of the invention. Variant nucleotide sequences include synthetically derived sequences, such as those generated for example, using site-directed mutagenesis. Generally, nucleotide sequence variants of the invention will have at least 40%, 50%, 60%, 70%, generally 80%, preferably 85%, 90%, and up to 95% or more sequence identity to its respective native nucleotide sequence. Thus, some fragments may also be variants.

"Variant" in the context of proteins is intended to mean a protein derived from the native protein by deletion or addition of one or more amino acids to the N-terminal and/or C-terminal end of the native protein: deletion or addition of one or more amino acids at one or more sites in the native protein: or substitution of one or more amino acids at one or more sites in the native protein. Such variants may result from, for example, genetic polymorphism or human manipulation. Conservative amino acid substitutions will generally result in variants that retain biological function. Variant proteins that retain a desired biological activity are encompassed within the subject invention. Variant proteins of the invention may include those that are altered in various ways including amino acid substitutions, deletions, truncations, and insertions. Methods for such manipulation are generally known in the art. See, for example, Kunkel (1985) *Proc. Natl. Acad. Sci. USA* 82:488-492; Kunkel et al. (1987) *Methods and Enzymol*; 154:367-382; and the references cited therein.

The methods of the invention are useful in any situation where increased expression of a nucleotide sequence is desired. Thus, the methods are useful for increasing the expression of endogenous as well as exogenous sequences. For example, for exogenous sequences, the booster sequence can be used to enhance expression of transgene-induced gene silencing. Therefore, the target sequence may be any nucleotide sequence of interest. In one embodiment, the methods can be used to produce peptides or proteins that cannot effectively be commercially produced by existing gene expression systems. For example, some proteins cannot be expressed in mammalian systems because the protein interferes with cell viability, cell proliferation, cellular differentiation, or protein assembly in mammalian cells. Such proteins include, but are not limited to, retinoblastoma protein, p53, angiostatin, and leptin. Likewise, the methods of the invention can be used to produce mammalian regulatory proteins. Other sequences of interest include proteins, hormones, growth factors, cytokines, preferably insulin, growth hormone, particularly human growth hormone, interferon, particularly α-interferon, β-glucocerebrosidase, serum albumin, particularly human serum albumin. hemoglobin, coliagen, etc. In such instances, generally, the booster sequence will be operably linked to a constitutive promoter.

In other instances, inducible promoters may be utilized. In one embodiment, the methods may be used to express disease and insect resistance genes in the plant. In this manner, enhancer sequences may be operably linked to a pathogen inducible promoter for enhanced disease resistance in a plant. It is recognized that some plant resistance is based on post-transcriptional gene silencing and such mechanisms may not work in the present invention. However, resistance mechanisms based on proteins would benefit the plant.

Insect resistance may be enhanced by expression of the booster sequence with a wound-inducible promoter such that the resistance genes, such as *Bacillus* toxins, would be expressed at the site of insect invasion. It is recognized that the pathogen resistance and insect resistance target sequences may be expressed by constitutive promoters. However, the use of an inducible promoter driving the booster sequence may benefit the plant from a yield and growth standpoint.

In another embodiment, the methods of the invention can be used to produce transgenic seed and seed products. In this manner, target sequences or genes of interest or, alternatively, the booster sequence can be operably linked with a seed-preferred, or endosperm promoter. Such seed proteins of interest include, but are not limited to, starches, storage proteins, proteins with enhanced nutritional value, specialty oils, carotenoids, etc.

Generally, the methods of the invention can be used for the increased expression of any target gene or sequence of interest, including therapeutic or immunogenic peptides and proteins, nucleic acids for controlling gene expression, genes to reproduce enzymatic pathways for chemical synthesis, genes to shunt an enzymatic pathway for enhanced expression of a particular intermediate or final product, industrial processes, and the like.

It is recognized that the methods of the invention can be used for enhanced expression in transformed plants, plant cells and tissues, seed, and the like. Thus, in some embodiments, it may be beneficial to provide the methods in a plant culture system for production of peptides or proteins of interest.

As discussed, a number of promoters can be used in the practice of the invention. The promoters can be selected based on the desired outcome. Generally, the booster sequence can be combined with promoters of choice to create increased expression of the target sequences in the tissue or organ of choice. However, in some instances, the targeting sequence may comprise a tissue, developmental, or inducible promoter for co-suppression and subsequent increased expression of the target sequence in particular tissues, organs, or developmental stages of the plant. Thus, the booster sequences can be combined with constitutive, tissue-specific, inducible, developmental, or other promoters for expression in plants depending upon the desired outcome.

Constitutive promoters include, for example. CaMV 35S promoter (Odell *et al*. (1985) *Nature* 313:810-812; rice actin (McElroy *et al*. (1990) *Plant Cell* 2:163-171; ubiquitin (Christensen *et al*. (1989) *Plant Mol. Biol*. 12:619-632 and Christensen *et al*. (1992) *Plant Mol. Biol*. 18:675-689); pEMU (Last *et al*. (1991) *Theor. Appl. Genet*. 81:581-588); MAS (Velten *et al*. (1984) *EMBO J.* 3:2723-2730); ALS promoter (U. S. Application Serial No. 08/409,297), and the like. Other constitutive promoters include those in U. S. Patent Nos. 5,608,149; 5,608,144; 5,604,121; 5,569,597; 5,466,785; 5,399,680; 5,268,463; and 5,608,142.

A number of inducible promoters are known in the art. For resistance genes, a pathogen- inducible promoter can be utilized. Such promoters include those from pathogenesis-related proteins (PR proteins), which are induced following infection by a pathogen; e.g., PR proteins, SAR proteins, beta-1,3-glucanase, chitinase, etc. See, for example, Redolfi *et al*. (1983) *Neth. J. Plant Pathol.* 89:245-254; Uknes *et al*. (1992) *Plant Cell* 4:645-656; and Van Loon (1985) *Plant Mol. Virol*. 4:111-116. Of particular interest are promoters that are expressed locally at or near the site of pathogen infection. See, for example, Marineau *et al*. (1987) *Plant Mol. Biol*. 9:335-342; Matton *et al*. (1989) *Molecular Plant-Microbe Interactions* 2:325-331; Somsisch *et al*. (1986) *Proc. Natl. Acad. Sci. USA* 83:2427-2430; Somsisch *et al*. (1988) *Mol. Gen. Genet.* 2:93-98; and Yang (1996) *Proc. Natl. Acad Sci. USA* 93:14972-14977. See also, Chen *et al*. (1996) *Plant J.* 10:955-966; Zhang *et al*. (1994) *Proc. Natl. Acad. Sci. USA* 91:2507-2511; Warner *et al*. (1993) *Plant J.* 3:191-201; Siebertz *et al*. (1989) *Plant Cell* 1:961-968; U. S. Patent No. 5,750,386; Cordero *et al*. (1992) *Physiol. Mol. Plant Path.* 41:189-200; and the references cited therein.

Additionally, as pathogens find entry into plants through wounds or insect damage, a wound-inducible promoter may be used in the DNA constructs of the invention. Such wound-inducible promoters include potato proteinase inhibitor (pin II) gene (Ryan (1990) *Ann. Rev. Phytopath*. 28:425-449; Duan *et al*. (1996) *Nature Biotechnology* 14:494-498; wun1 and wun2, U. S. Patent No. 5,428,148; win 1 and win2 (Stanford *et al*. (1989) *Mol. Gen. Geuet*. 215:200-208); systemin (McGurl *et al*. (1992) *Science* 225:1570-1573); WIP1 (Rohmeier *et al*. (1993) *Plant Mol. Biol.* 22:783-792: Eckelkamp *et al*. (1993) *FEBS Letters* 323:73-76); MPI gene (Corderok *et al*. (1994) *Plant J.* 6(2):141-150; and the like. Such references are herein incorporated by reference.

Chemical-regulated promoters can be used to modulate the expression of a gene in a plant through the application of an exogenous chemical regulator. Depending upon the objective, the promoter may be a chemical-inducible promoter, where application of the chemical induces gene expression; or a chemical-repressible promoter, where application of the chemical represses gene expression. Chemical-inducible promoters are known in the art and include, but are not limited to, the maize In2-2 promoter, which is activated by benzenesulfonamide herbicide safeners: the maize GST promoter, which is activated by hydrophobic electrophilic compounds that are used as preemergent herbicides: and the tobacco PR-1a promoter, which is activated by salicylic acid. Other chemical-regulated promoters of interest include steroid-responsive promoters (see, for example, the glucocorticoid-inducible promoter in Schena *et al*. (1991) *Proc. Natl. Acad. Sci USA* 88:10421-10425 and McNellis *et al*. (1998) *Plant J*. 14(2):247-257), and tetracycline-inducible and tetracycline-repressible promoters (see, for example, Gatz *et al*. (1991) *Mol. Gen. Genet*. 227:229-237, and U. S. Patent Nos. 5,814,618 and 5,789,156), herein incorporated by reference.

Where enhanced expression in particular tissues is desired, tissue-specific promoters can be utilized. Tissue-specific promoters include those taught in Yamamoto *et al*. (1997) *Plant J*. 12(2)255-265; Kawamata *et al*. (1997) *Plant Cell Physiol.* 38(7):792-803: Hansen *et al*. (1997) *Mol. Gen Genet*. 254(3):337-343; Russel *et al*. (1997) *Transgenic Res.* 6(2):157-168; Rinehart et al. (1996) *Plant Physiol*. 112(3):1331-1341; Van Camp *et al*. (1996) *Plant Physiol.* 112(2):525-535; Canevasini et al. (1996) *Plant Physiol.* 112(2):513-524; Yamamoto *et al*. (1994) *Plant Cell Physiol.* 35(5):773-778; Lam (1994) *Results Probl. Cell Differ*. 20:181-196; Orozco *et al*. (1993) *Plant Mol. Biol*. 23(6):1129-1138; Matsuoka *et al*. (1993) *Proc Natl. Acad. Sci.* USA 90(20):9586-9590; and Guevara-Garcia *et al*. (1993) *Plant J.* 4(3):495-505.

Leaf- specific promoters can similarly be used if desired, and are taught in references which include Yamamoto *et al*. (1997) *Plant J*. 12(2):255-265; Kawamata *et al*. (1997) *Plant Cell Physiol*, 38(7):792-803; Hansen *et al*. (1997) *Mol. Gen. Genet*. 254(3):337-343; Russel *et al*. (1997) *Transgenic Res.* 6(2):157-168; Rinehart *et al*. (1996) *Plant Physiol*. 112(3):1331-1341; Van Camp et al. (1996) *Plant Physiol*. 112(2):525-535: Canevascini *et al.* (1996) *Plant Physiol.* 112(2):513-524; Yamamoto *et al.* (1994) *Plant Cell Physiol.* 35(5)773-778: Lam (1994) *Results Probl. Cell Differ*. 20:181-196; Orozco *et al.* (1993) *Plant Mol. Biol.* 23(6):1129-1138; Matsuoka *et al*. (1993) *Proc. Natl. Acad. Sci* USA:90(20) 9586-9590; and Guevara-Garcia *et al*. (1993) *Plant J*. 4(3):495-505.

Root-specific promoters are known and can be selected from the many available from the literature. See, for example, Hire *et al.* (1992) *Plant Mol. Biol*. 20(2):207-218 (soybean root-specific glutamine synthetase gene); Keller and Baumgartner (1991) *Plant Cell* 3(10):1051-1061 (root-specific control element in the GRP 1.8 gene of French bean); Sanger *et al*. (1990) *Plant Mol. Biol*. 14(3):433-443 (root-specific promoter of the mannopine synthase (MAS) gene of *Agrobacterium tumefaciens*) Miao *et al.* (1991) *Plant Cell* 3(1):11-22 (full-length cDNA clone encoding cytosolic glutamine synthetase (GS), which is expressed in roots and root nodules of soybean). Bogusz *et al*. (1990) *Plant Cell* 2(7):633-641 (root specific promoters from hemoglobin genes from the nitrogen-fixing nonlegume *Parasponia andersonii* and the related non-nitrogen-fixing nonlegume *Trema tomeniosa:* Leach and Aoyagi (1991) *Plant Science* (Limerick) 79(1):69-76 (rolC and rolD root-including genes of *Agrobacterium rhizogenes);* Teeri *et al*. (1989) *EMBO J.* 8(2):343-350 (octopine synthase and TR2' gene); (VfENOD-GRP3 gene promoter); Kuster *et al*. (1995) *Plant Mol. Biol*. 29(4):759-772 and Capana *et al.* (1994) *Plant Mol. Biol*. 25(4):681-691 (rolB promoter). See also U. S. Patent Nos. 5,837,876; 5,750,386; 5,633,363; 5,459,252; 5,401,836; 5,110,732; and 5,023,179.

"Seed-preferred" promoters include both "seed-specific" promoters (those promoters active during seed development such as promoters of seed storage proteins) as well as "seed-germinating" promoters (those promoters active during seed germination). See Thompson *et al.* (1989) *Bioassays* 10:108, herein incorporated by reference. Such seed-preferred promoters include, but are not limited to, Cim1 (cytokinin-induced message); cZ10B1 (Maize 19 kDa zein); celA (cellulose synthase); gama-zein; Glob-1; bean β-phaseolin; napin; β-conglycinin; soybean lectin; cruciferin; maize 15 kDa zein; 22 kDa zein; 27 kDa zein; g-zein; waxy; shrunken 1; shrunken 2; globulin 1; etc.

The booster sequences of the invention may be provided in DNA constructs or expression cassettes for expression in the plant of interest. The cassette will include 5' and 3' regulatory sequences operably linked to a booster sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame.

The expression cassette may additionally contain at least one additional gene to be cotransformed into the organism. Alternatively, the additional gene(s) can he provided on multiple expression cassettes.

Such an expression cassette is provided with a plurality of restriction sites for insertion of the sequences of the invention to be under the transcriptional regulation of the regulatory regions. The expression cassette may additionally contain selectable marker genes.

The expression cassette will include in the 5'-3' direction of transcription, a transcriptional and translational initiation region, a DNA sequence of the invention, and a transcriptional and translational termination region functional in plants. The transcriptional initiation region, the promoter, may be native or analogous, or foreign or heterologous, to the plant host. Additionally, the promoter may be the natural sequence or alternatively a synthetic sequence. By "foreign" is intended that the transcriptional initiation region is not found in the native plant into which the transcriptional initiation region is introduced. As used herein, a chimeric gene comprises a coding sequence operably linked to a transcriptional initiation region that is heterologous to the coding sequence.

The termination region may be native with the transcriptional initiation region, may be native with the operably linked DNA sequence of interest, or may be derived from another source. Convenient termination regions are available from the Ti-plasmid ofA. *tumefaciens,* such as the octopine synthase and nopaline synthase termination regions. See also Guerineau *et al*. (1991) *Mol. Gen. Genet*. 262:141-144; Proudfoot (1991) *Cell* 64:671-674; Sanfacon *et al.* (1991) *Genes Dev.* 5: 141-149; Mogen *et al*. (1990) *Plant Cell* 2:1261-1272; Munroe *et al*. (1990) Gene 91:151-158; Ballas *et al*. (1989) *Nucleic Acids Res.* 17:7891-7903; and Joshi *et al.* (1987) *Nucleic Acid Res*. 15:9627-9639.

Where appropriate, the gene(s) for which expression is to be enhanced may be optimized for expression in the transformed plant. That is, the genes can be synthesized using plant-preferred codons corresponding to the plant of interest. Methods are available in the art for synthesizing plant-preferred genes. See, for example, U. S. Patent Nos. 5,380,831 and 5,436,391, and Murray *et al*. (1989) *Nucleic Acids Res.* 17:477-498, herein incorporated by reference.

The expression cassettes may additionally contain 5' leader sequences in the expression cassette construct. Such leader sequences can act to enhance translation. Translation leaders are known in the art and include: picornavirus leaders, for example, EMCV leader (Encephalomyocarditis 5' noncoding region), Elroy-Stein *et al*. (1989) *PNAS USA* 86:6126-6130; potyvirus leaders, for example, TEV leader (Tobacco Etch Virus), Allison *et al*. (1986); MDMV Leader (Maize Dwarf Mosaic Virus), *Virology* 154:9-20; human immunoglobulin heavy-chain binding protein (BiP). Macejak *et al*. (1991) *Nature* 353:90-94; untranslated leader from the coat protein mRNA of alfalfa mosaic virus (AMV RNA 4). Jobling *et al.* (1987) *Nature* 325:622-625; tobacco mosaic virus leader (TMV), Gallie *et al*. (1989) in *Molecular Biology of RNA,* ed. Cech (Liss. New York), pp. 237-256; and maize chlorotic mottle virus leader (MCMV), Lommel *et al*. (1991) *Virology* 81:382-385. See also, Della-Cioppa *et al.* (1987) *Plant Physiol.* 84:965-968. Other methods known to enhance translation can also be utilized, for example, introns, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions. e.g., transitions and transversions, may be involved.

Plants transformed with a DNA construct of the invention may be produced by standard techniques known in the art for the genetic manipulation of plants. DNA can be transformed into plant cells using any suitable technology, such as a disarmed Ti-plasmid vector carried by *Agrobacterium* exploiting its natural gene transferability (EP-A-270355; EP-A-0116718; *NAR* 12(22):87211-87215 (1984); Townsend *et al*., U. S. Patent No. 5,563,055); particle or microprojectile bombardment (U. S. Patent No. 5,100,792; EP-A-444882; EP-A-434616; Sanford *et al*. U. S. Patent No. 4,945,050; Tomes *et al*. (1995) "*Direct DNA Transfer Into Plant Cells via Microprojectile Bombardment*," in *Plant Cell, Tissue, and Organ Culture: Fundamental Methods*, ed. Gamborg and Phillips (Springer-Verlag, Berlin); and McCabe *et al.* (1988) *Biotechnology* 6:923-926); microinjection (WO 92/09696; WO 94/00583; EP 331083; EP 175966; Green *et al*. (1987) *Plant Tissue and Cell Culture*, Academic Press; Crossway *et al.* (1986) *Biotechniques* 4:320-334); electroporation (EP 290395; WO 8706614; Riggs *et al.* (1986) *Proc. Nat. Acad Sci. USA* 83:5602-5606; D'Halluin (1992) *Plant Cell* 4:1495-1505); other forms of direct DNA uptake (DE 4005152; WO 9012096; U. S. Patent No. 4,684,611; Paszkowski *et al*. (1984) *EMBO J*. 3:2717-2722); liposome-mediated DNA uptake (*e.g*., Freeman *et al*. (1984) *Plant Cell Physiol*. 29:1353); or the vortexing method (*e.g*., Kindle (1990) *Proc. Nat. Acad. Sci USA* 87:1228). Physical methods for the transformation of plant cells are reviewed in Oard (1991) *Biotech. Adv*. 9:1-11. See generally, Weissinger *et al*. (1988) *Ann. Rev. Genet.* 22:421-477: Sanford *et al*. (1987) *Particulate Science and Technology* 5:27-37; Christou *et al*. (1988) *Plant Physiol*. 87:671-674; McCabe *et al.* (1988) *Bio*/*Technology* 6:923-926; Finer and McMullen (1991) *In Vitro Cell Dev. Biol*. 27P:175-182; Singh *et al*. (1998) *Theor. Appl. Genet.* 96:319324; Datta *et al*. (1990) *Biotechnology* 8:736-740; Klein *et al*. (1988) *Pro. Natl. Acad. Sci. USA* 85:4305-4309; Klein *et al*. (1988) *Biotechnology* 6:559-563; Tomes, U. S. Patent No. 5,240,855; Buising *et al*., U. S. Patent Nos. 5.322.783 and 5,324,646; Klein *et al.* (1988) *Plant Physiol.* 91:440-444: Fromm *et al*. (1990) *Biotechnology* 8:833-839; Hooykaas-Van Slogteren *et al*. (1984) *Nature* (London) 311:763-764; Bytebier *et al*. (1987) *Proc. Natl. Acad. Sci. USA* 84:5345-5349; De Wet *et al*. (1985) in *The Experimental Manipulation of Ovule Tissues*, ed. Chapman *et al*. (Longman, New York), pp. 197-209; Kaeppler *et al*. (1990) *Plant Cell Reports* 9:415-418 and Kaeppler *et al*. (1992) *Theor. Appl. Genet*. 84:560-566; Li *et al.* (1993) *Plant Cell Reports* 12:250-255; Christou and Ford (1995) *Annals of Botany* 75:407-413; and Osjoda *et al*. (1996) *Nature Biotechnology* 14:745-750; all of which are herein incorporated by reference.

*Agrobacterium* transformation is widely used by those skilled in the art to transform dicotyledonous species. Recently, there has been substantial progress towards the routine production of stable, fertile transgenic plants in almost all economically relevant monocot plants (Toriyama *et al*. (1988) *Bio*/*Technology* 6:1072-1074; Zhang, *et al*. (1988) *Plant Cell Rep.* 7:379-384; Zhang *et al*. (1988) *Theor. Appl. Genet*. 76:835-840; Shimamoto *et al.* (1989) *Nature* 338:274-276; Datta *et al*. (1990) *Bio*/*Technology* 8:736-740; Christou *et al*. (1991) *Biotechnology* 9:957-962: Peng *et al*. (1991) International Rice Research Institute, Manila, Philippines, pp. 563-574; Cao *et al*. (1992) *Plant Cell Rep.* 11:585-591; Li *et al*. (1993) *Plant Cell Rep.* 12:250-255; Rathore *et al*. (1993) *Plant Mol. Biol*. 21:871-884; Fromm *et al*. (1990) *Bio*/*Technology* 8:833-839: Gordon-Kamm *et al*. (1990) *Plant Cell* 2:603-618; D'Halluin *et al.* (1992) *Plant Cell* 4:1495-1505; Walters *et al*. (1992) *Plant Mol. Biol*. 18:189-200; Koziel *et al*. (1993) *Biotechnology* 11:194-200; Vasil, I.K. (1994) *Plant Mol. Biol*. 25:925-937; Weeks *et al.* (1993) *Plant Physiol*. 102:1077-1084; Somers *et al*. (1992) *Bio*/*Technology* 10:1589-1594; WO 92/14828). In particular, *Agrobacterium* mediated transformation is now emerging also as a highly efficient transformation method in monocots (Hiei, *et al*. (1994) *The Plant Journal* 6:271-282). See also, Shimamoto, K. (1994) *Current Opinion in Biotechnology* 5:158-162; Vasil, *et al*. (1992) *Bio*/*Technology* 10:667-674; Vain, *et al*. (1995) *Biotechnology Advances* 13(4):653-671; Vasil *et al*. (1996) *Nature Biotechnology* 14:702.

Microprojectile bombardment, electroporation, and direct DNA uptake are preferred where *Agrobacterium* is inefficient or ineffective. Alternatively, a combination of different techniques may be employed to enhance the efficiency of the transformation process, e.g., bombardment with *Agrobacterium*-coated microparticles (EP-A-486234) or microprojectile bombardment to induce wounding followed by co-cultivation with *Agrobacterium* (EP-A-486233).

Following transformation, a plant may be regenerated, e.g., from single cells, callus tissue or leaf discs, as is standard in the art. Almost any plant can be entirely regenerated from cells, tissues, and organs of the plant. Available techniques are reviewed in Vasil *et al*. (1984) in *Cell Culture and Somatic Cell Genetics of Plants, Vols. I, II, and III, Laboratory Procedures and Their Applications* (Academic press); and Weissbach *et al*. (1989) *Methods for Plant Mol. Biol*.

The transformed plants may then be grown, and either pollinated with the same transformed strain or different strains, and the resulting hybrid having expression of the desired phenotypic characteristic identified. Two or more generations may be grown to ensure that expression of the desired phenotypic characteristic is stably maintained and inherited, and then seeds harvested to ensure expression of the desired phenotypic characteristic has been achieved.

The particular choice of a transformation technology will be determined by its efficiency to transform certain plant species as well as the experience and preference of the person practicing the invention with a particular methodology of choice. It will be apparent to the skilled person that the particular choice of a transformation system to introduce nucleic acid into plant cells is not essential to or a limitation of the invention, nor is the choice of technique for plant regeneration.

Also according to the invention, there is provided a plant cell having the constructs of the invention. A further aspect of the present invention provides a method of making such a plant cell involving introduction of a vector including the construct into a plant cell. For integration of the construct into the plant genome, such introduction will be followed by recombination between the vector and the plant cell genome to introduce the sequence of nucleotides into the genome. RNA encoded by the introduced nucleic acid construct may then be transcribed in the cell and descendants thereof, including cells in plants regenerated from transformed material. A gene stably incorporated into the genome of a plant is passed from generation to generation to descendants of the plant, so such descendants should show the desired phenotype.

The present invention also provides a plant comprising a plant cell as disclosed. Transformed seeds and plant parts are also encompassed.

In addition to a plant, the present invention provides any clone of such a plant, seed, selfed or hybrid descendants, and any part of any of these, such as cuttings or seed. The invention provides any plant propagule, that is any part which may be used in reproduction or propagation, sexual or asexual, including cuttings, seed, and so on. Also encompassed by the invention is a plant which is a sexually or asexually propagated off-spring, clone, or descendant of such a plant; or any part or propagule of said plant, off-spring, clone, or descendant. Plant extracts and derivatives are also provided.

The present invention may be used for transformation of any plant species, including, but not limited to, corn *(Zea mays*), canola *(Brassica napus, Brassica rapa* ssp.), alfalfa *(Medicago sativa*), rice (*Oryza sativa*), rye *(Secale cereale*), sorghum (*Sorghum bicolor, Sorghum vulgare*), sunflower *(Helianthus annuus),* wheat (*Triticum aestivum*), soybean (*Glycine max*), tobacco (*Nicotiana tabacum*), potato *(Solanum tuberosum*), peanuts *(Arachis hypogaea*), cotton (*Gossypium hirsutum*), sweet potato *(Ipomoea batatus*), cassava (*Manihot esculenta*), coffee (*Cofea* ssp.), coconut *(Cocos nucifera*), pineapple (*Ananas comosus*), citrus trees *(Citrus* spp.), cocoa (*Theobroma cacao),* tea *(Camellia sinensis*), banana *(Musa* spp.), avocado *(Persea americana*), fig (*Ficus casica),* guava (*Psidium guajava*), mango *(Mangifera indica*), olive *(Olea europaea*), papaya *(Carica papaya),* cashew (*Anacardium occidental),* macadamia (*Macadamia integrifolia*), almond (*Prunus amygdalus*), sugar beets *(Beta vulgaris),* oats, barley, vegetables, ornamentals, and conifers.

Preferably, plants of the present invention are crop plants (for example, cereals and pulses, maize, wheat, potatoes, tapioca, rice, sorghum, millet, cassava, barley, pea, and other root, tuber, or seed crops. Important seed crops are oil-seed rape, sugar beet, maize, sunflower, soybean, and sorghum. Horticultural plants to which the present invention may be applied may include lettuce; endive; and vegetable brassicas including cabbage, broccoli, and cauliflower; and carnations and geraniums. The present invention may be applied to tobacco, cucurbits, carrot, strawberry, sunflower, tomato, pepper, chrysanthemum, poplar, eucalyptus, and pine.

Grain plants that provide seeds of interest include oil-seed plants and leguminous plants. Seeds of interest include grain seeds, such as corn, wheat, barley, rice, sorghum, rye, etc. Oil seed plants include cotton, soybean, safflower, sunflower, Brassica, maize, alfalfa, palm, coconut, etc. Leguminous plants include beans and peas. Beans including guar, locust bean, fenugreek, soybean, garden beans, cowpea, mungbean, lima bean, fava bean, lentils, chickpea, etc.

Since the effect of HC-Pro on suppression of PTGS is clearly protein-mediated rather than RNA-mediated (Anandalakshmi *et al.,* 1998: Brigneti *et al*., 1998), we used protein-protein interaction assays to search for plant proteins that interact with HC-Pro. Using HC-Pro as bait in a yeast two-hybrid system, we found four HC-Pro-interacting plant proteins. Our overall approach has been to overexpress or interfere with the expression of these proteins and then assay the effect of the manipulations on gene silencing and its modulation by HC-Pro. These methods have already successfully identified one of the HC-Pro-interactors as a plant regulator of silencing. The plant regulator of silencing is a novel calmodulin-related protein (rgs-CaM). This cellular protein, like HC-Pro itself (Brigneti *et al*., 1998), reverses PTGS when expressed from a viral vector and interferes with the initiation of virus-induced gene silencing when expressed ectopically in transgenic plants.

The silencing suppressor activity of HC-Pro can be separated from its detrimental developmental phenotype. Two observations indicate that this is the case. Several transgenic tobacco lines that express mutant versions of P1/HC-Pro are available. One of these mutant lines does not have the usual "HC-Pro" developmental phenotype. It looks completely normal and grows at a normal rate. When crossed with a GUS-silenced line, it was noted that the mutant P1/HC-Pro failed to lift GUS silencing in the vascular tissue of the offspring, but did so in all other tissues of the plant Thus, tissue specific expression of HC-Pro or rgs-CaM can yield plants that suppress PTGS but are phenotypically normal.

The role of rgs-CaM in regulating PTGS allows manipulation of this pathway, which can be exploited in two different ways. 1) Suppression of the pathway (for example by over expression of rgs-CaM) allows stable high level expression of beneficial transgenes in plants. 2) In some cases; PTGS is used to eliminate the expression of detrimental or unwanted genes. In those cases, it is possible to use rgs-CaM to enhance PTGS and thus stabilize the silenced phenotype desired in these applications. HC-Pro is a multifunctional protein impacting pathways that affect both developmental and defense pathways in plants. Since the effects of HC-Pro on plant defense and development are mediated by interactions with cellular proteins that normally regulate these processes, it.is possible to use the HC-Pro-interacting proteins such as rgs-CaM to engineer plants to express only one aspect of the "HC-Pro" phenotype.

By cloning mutant versions of rgs-CaM into a PVX vector and assaying the ability of the construct to reverse PTGS. one can assay the activity of rgs-CaM variants. This reversal of gene silencing assay is currently in use in the lab and was used to demonstrate that rgs-CaM is a regulator of PTGS

Following are examples which illustrate procedures for practicing the invention. These examples should not be construed as limiting. All percentages are by weight and all solvent mixture proportions are by volume unless otherwise noted.

### Example 1 - Isolation of cDNAs Encoding HC-Pro Interacting Proteins from Tobacco

In order to identify HC-Pro-interacting plant proteins, we screened a yeast two-hybrid tobacco cDNA library (kindly provided by Dr. Chris Mau, UG Dsvis) using the tobacco etch viral HC-Pro as bait. Two million cDNAs were screened and sixteen HC-Pro-interacting tobacco cDNAs were isolated on the basis of ability to grow in the absence of histidine and to activate the lacZ gene. Eight of these tobacco cDNAs met the following criteria. They did not interact with empty bait or negative control (lamin) plasmids and, when cloned into the DNA binding domain fusion plasmid, they interacted positively with HC-Pro in the activation domain fusion plasmid (reciprocal positives). The cDNAs for these eight putative HC-Pro interacting proteins were sequenced and comparative sequence analyses were carried out using the programs BLAST and FASTA. The cDNA encoding rgs-CaM was identified [SEQ ID NO: 1]. Based on this sequence, rgs-CaM homologues from other species have been identified and can be used according to the teachings herein, including tomato [GENBANK accession #A1487362], soybean [GENBANK accession #AW350323], and *Arabidopsis* [GENBANK accession #AB016888. translation of nucleotides 22076 through 21546].

HC-Pro-interacting protein B-3 (rgs-CaM) [SEQ ID NO: 2] shows high homology to calmodulins and related calcium binding proteins at its C-terminus, but with a 50 amino acid extension at its N-terminus. Subsequent experiments with B-3 led to the discovery that it acts as a regulator of gene silencing and these results are discussed in more detail in the following examples. Since the B-3 protein is a novel calmodulin-related protein and it regulates gene silencing, we named it rgs-CaM. for regulator of gene silencing (rgs), calmodulin-related protein (CaM).

Calmodulin (CaM) is a major intracellular receptor for the second messenger Ca²⁺ and is expressed in every cell of all eukaryotes (Rudd and Franklin-Tong, 1999; Snedden and Hillel, 1999). It is a signaling molecule which has no enzymatic activity of its own, but binds to Ca²⁺ and changes conformation allowing it to bind to specific target proteins and alter their activity. In general. CaM binds to and masks an autoinhibitory domain on the target protein and thereby activates it. All plants appear to have calmodulins that resemble the mammalian calmodulin. However, recent studies have identified a family of calmodulin-related proteins in plants distinct from the generic caimoduiin in mammais. All these CaM and CaM-like molecules contain a common structural motif called the "EF hand", a helix-loop-helix structure that binds to a single Ca²⁺ ion. Mammalian CaM has four EF hands, while CaM-related proteins of plants have varying numbers of this motif ranging from three to six, and many have either C- or N-terminal extensions as compared to CaM. Plant CaM-related proteins appear to differ in their ability to activate known CaM targets, and it is thought that there might be target specificity for different members of the plant CaM superfamily. Full-length native rgs-CaM has three EF hands and an N-terminal extension of about 40-50 amino acids that may specify the intracellular location or regulatory properties of rgs-CaM. The following examples disclose a variety of biochemical and genetic approaches to demonstrate the activity of the protein and how it transduces a signal that affects gene silencing.

### Example 2 - Stable transformation experiments using the rgs-CaM cDNAs

To demonstrate the role of rgs-CaM we overexpress or interfere with the expression of rgs-CaM cDNAs using stable transformation techniques. An alternative approach using potato virus X (PVX) as a vector to express high levels of rgs-CaM was pursued simultaneously.

Initial stable transformation experiments use cDNAs for rgs-CaM under control of the strong cauliflower mosaic virus 35S promoter for stable transformation of both *Nicotiana tabacum* (tobacco) and *N. benthamiana* (a species related to tobacco that is well suited for experiments using viral vectors). Greater than 30 individual primary transformants in both tobacco species are obtained. The primary transformants are selfed and are analyzed for segregation of the transgene (initially inferred by selection on kanamycin). Lines that segregate 3:1 for kanamycin resistance and also have a visible phenotype are selected for further studies. The selected lines are analyzed by Southern blot analysis to determine the number of transgenes, by Northern blot analysis to determine the level of expression of the transgene (and thus determine if the transgene is overexpressed or silenced) and for changes in gene silencing characteristics using a number of assays.

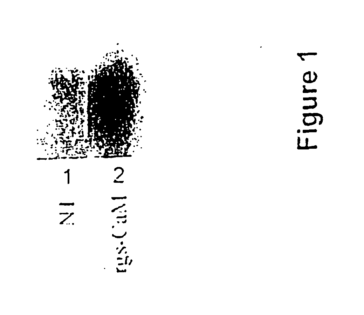

Interestingly, rgs-CaM cDNAs confer a characteristic phenotype in a number of the *N. benthamiana* primary transformants. The phenotypes of these transgenic lines might be conferred either by increased expression or by reduced expression of the particular transgene. Reduced expression of a transgene generally occurs in some fraction of transgenic lines due to either transcriptional or post-transcriptional gene silencing. The phenotype of rgs-CaM primary transformants in *N. benthamiana* is particularly interesting because it mimics that of plants transformed with HC-Pro. These rgs-CaM transgenic plants are found to greatly overexpress rgs-CaM in RNA as compared to non-transgenic controls. See Figure 1, lanes 1 and 2. This indicates that rgs-CaM is a plant suppressor of PTGS.

### Example 3 - rgs-CaM is a Plant Regulator of Post-Transcriptional Gene Silencing

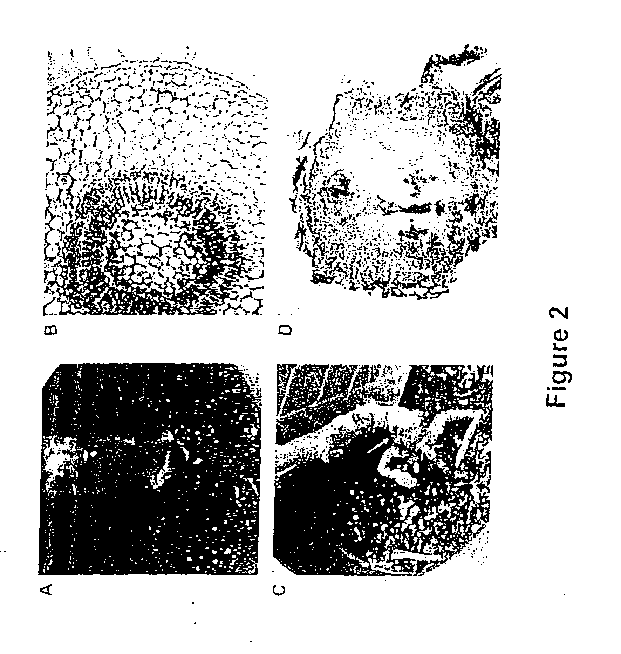

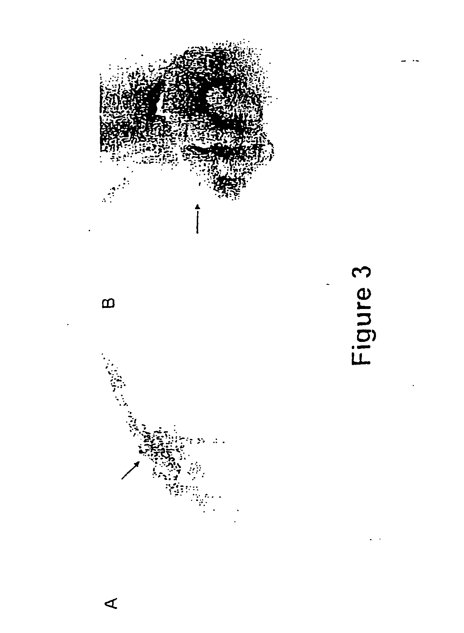

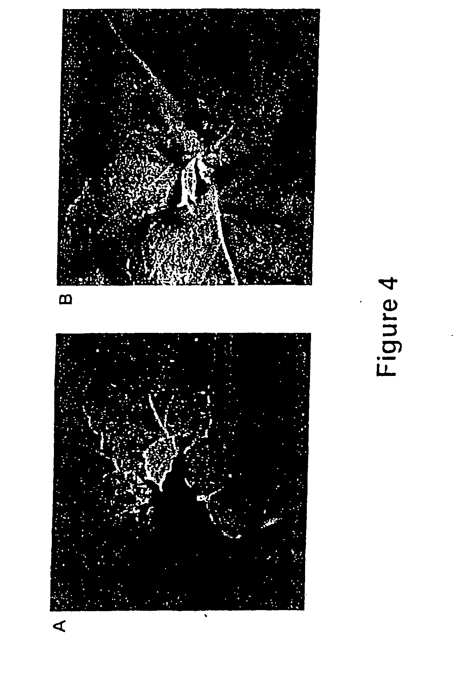

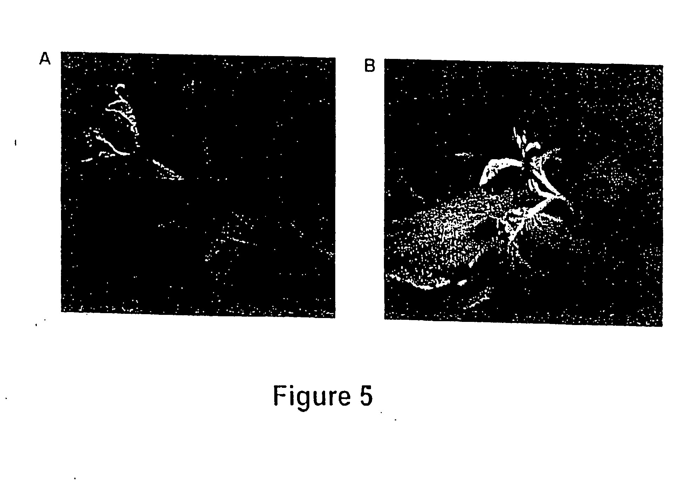

The phenotype of a number of independent rgs-CaM expressing transgenic I ines is found to mimic that seen in P1/HC-Pro transgenic lines, and this surprising finding indicates that rgs-CaM, like P1/HC-Pro, acts as a regulator of silencing. We use two different assays to directly demonstrate the ability of this protein to affect gene silencing. There are four lines of evidence that rgs-CaM regulates PTGS and these are described below:
1) The phenotype of rgs-CaM transgenic lines mimics that of transgenic plants expressing the HC-Pro suppressor of silencing. Expression of P1/HC-Pro in transgenic plants confers several unique developmental characteristics, the most prominent of which are slow growth with reduced root biomass and development of a tumor at the junction of the stem and the root (compare the tumor in Fig. 2C to normal stem/root junction in Fig. 2A). The tumor associated with expression of PI/HC-Pro has a differentiated cytology (i.e. all the appropriate differentiated cell types are present, but the cell types are improperly arranged) (compare the aberrant organization of cells in Fig. 2D to the normal organization shown in Fig. 2B).
   Several of the *N. benthamiana* rgs-CaM primary transformants develop a phenotype reminiscent of that seen in *N. tabacum* plants expressing the potyviral P1/HC-Pro suppressor of gene silencing. They grow slowly and have a tumorous growth which develops at the junction of the stem and the root (Fig. 3). Like the HC-Pro transgenic lines, these rgs-CaM tumors are composed of differentiated cells that are aberrantly arranged (data not shown). These results suggest that post-transcriptional gene silencing is used during normal plant development and that suppression of this mechanism produces developmental anomalies. The fact that rgs-CaM transgenic lines have a phenotype similar to that seen in transgenics expressing the viral suppressor indicates that rgs-CaM is a cellular regulator of PTGS and that this protein acts, at least in part, to mediate the suppressor activity of HC-Pro.
2) An rgs-CaM transgenic line is disabled for virus-induced gene silencing. To directly test if the rgs-CaM transgenic line is altered in the regulation of post-transcriptional gene silencing, we use a virus-induced gene silencing (VIGS) assay. This assay is well established (Baulcombe, 1999; Jones *et al*., 1998; Ruiz *et al*., 1998) and has been used successfully in our lab (Anandalakshmi *et al*., 1998). In this assay, a transgenic plant line expressing high levels of GFP is infected with a PVX vector expressing the GFP sequence (other reporter genes could be used so long as the gene is the same in the transgenic line and in the viral vector). The PVX-GFP replicates and moves systemically through the plant expressing high levels of GFP in leaves invaded early in the infection. The infection process triggers VIGS. a kind of post-transcriptional gene silencing. First the GFP transgene is silenced locally, and then throughout the upper part of the plant. Finally, very late in the infection, the viral vector (PVX-GFP) becomes silenced and is eliminated from the plant. At this point, in the absence of the viral trigger, the GFP transgene turns back on. We have previously shown that P1/HC-Pro interferes with VIGS in this system (Anandalakshmi *et al*., 1998). To demonstrate the effect of rgs-CaM in the same system, we cross an rgs-CaM expressing line (line 19, an rgs-CaM line with a pronounced phenotype, a primary transformant as described in Example 2) with a line expressing high levels of GFP (line 16C, the kind gift ofD. Baulcombe, Sainsbury labs, Norwich, UK) to produce plants hemizygous for both the GFP and rgs-CaM transgenes. Plants from both lines are infected with PVX-GFP and are capable of supporting replication and spread of this virus based on local infection foci and systemic symptoms. The hemizygous 16C GFP plants (without the rgs-CaM transgene) become completely silenced for the GFP transgene at about ten days post-inoculation with PVX-GFP (and therefore the upper part of the plant fluoresces red under UV light due to the presence of chlorophyll rather than green as it does in the presence of GFP; Fig. 4A), as well as by a dramatic reduction in both GFP-transgene RNA and PVX-GFP viral RNA (data not shown). In contrast, plants hemizygous for both the GFP and rgs-CaM transgenes fail to silence when infected with PVX-GFP and continue to fluoresce green under long wave UV light (Fig. 4B). This is a direct demonstration that the rgs-CaM transgenic line is deficient in PTGS.
3) rgs-CaM reverses gene silencing when expressed from a PVX vector. To further demonstrate the role of rgs-CaM in regulating gene silencing, we employ a reversal of silencing assay described by Brigneti *et al*. (1998). In this approach, an *N. benthamiana* transgenic line (line 16C) expressing high levels of green fluorescent protein (GFP) is post-transcriptionally silenced by infiltrating the leaves with *Agrobacterium tumefaciens* carrying a vector that also expresses GFP. The Agrobacterium does not move from the inoculated leaf, but it induces PTGS of the GFP transgene locally, and then the GFP silencing spreads systemically through the plant until it is completely silenced. The GFP silencing in these plants can be reversed by infection with a PVX vector expressing a viral suppressor of silencing such as the potyviral HC-Pro or the cucumber mosaic virus 2a gene product (Brigneti *et al*., 1998). We silence the GFP plants by Agro-infiltration and then infect them with potato virus X carrying either wild-type HC-Pro (PVX-HC), a mutant inactive version of HC-Pro (PVX-noHC), or the rgs-CaM cDNA sequence (PVX-rgs-CaM). Plants infected with each of these viruses develop symptoms typical of the particular PVX vector (a mild mosaic pattern on leaves for PVX-rgs-CaM and PVX-noHC; a systemic necrosis for PVX-HC). The plants infected with PVX-noHC remain silenced for the GFP transgene (and therefore red under UV light, Fig. 5A). In contrast, plants infected with PVX-rgs-CaM show a reversal of the GFP silencing in the systemically infected portion of the previously silenced plant (areas of green in the red background, Fig. 5B). This reversal of silencing is typical of viral suppressors of silencing expressed from PVX (Brigneti *et al.,* 1998) and is observed in the portions of silenced plants systemically infected with PVX-HC (data not shown). This result is consistent with the VIGS experiment described above and confirms that rgs-CaM is a cellular regulator of PTGS.

### Example 4

The ability of rgs-CaM over expressing transgenic plants to suppress transgene-induced gene silencing is demonstrated in *Agrobacterium* infiltration studies. *A Nicotiana benthamiana* transgenic line expressing both rgs-CaM and green fluorescent protein (GFP) or a control line expressing only the GFP transgene are infiltrated with a culture of *Agrobacterium* that also expresses the GFP transgene. Silencing of the GFP transgene in control plants is apparent in the region of local infiltration within a few days. Within two weeks this transgene-indued gene silencing moves throughout the upper part of the plant resulting in complete systemic silencing of the plant. In contrast, the rgs-CaM-expressing plants fail to silence in response to infiltration with *Agrobacterium*, or silencing occurs initially in a small local area but fails to spread efficiently. This result indicates that rgs-CaM is a suppressor of transgene induced gene silencing.

### Example 5

In order to determine whether the booster sequence will function to enhance expression of a stably incorporated transgene, the present Example may be conducted. A plant is first transformed by any mode of stable transformation with an endogenous plant gene for the purpose of over expressing that particular gene. A portion of the transformants are cosuppressed for the introduced gene and would fail to express gene product. These cosuppressed plants are then crossed with a plant stably transformed with the booster sequence, such as the transformants described in Example 2. The offspring of the cross express the previously silenced (cosuppressed) introduced endogenous gene.

### Example 6

The following Example describes the present method being utilized to express high levels of an endogenous gene product. A plant stably transformed with one or more copies of the booster sequence such as the rgs-CaM transformed plants described in Example 2 herein is subsequently transformed with an additional copy or additional copies of an endogenous gene for the purpose of overexpressing that gene product. The transformants display high levels of expression of the introduced gene product and/or reduced numbers of cosuppressed offspring that fail to express the gene product.

There are several other examples of transgene-induced post-transcriptional silencing where the booster sequence is expected to interfere with gene silencing and thereby boost expression. Two examples of such lines are currently being assayed by crossing with the P 1/HC-Pro expressing transgenic lines and assaying for reversal of gene silencing. These silenced lines were both constructed in France at the INRS in the lab of Herve Vaucheret.

Transgenic line 475-2.1 is a tobacco transgenic line and is post-transcriptionally silenced for both transgene encoded and endogenous nitrite reductase (Nii) genes and has been previously described (J.C. Palauqui, T. Elmayan, F. Dorlhac de Borne, P. Crete, C. Charles and H. Vaucheret (1996) "Frequencies, Timing, and Spatial Patterns of Co-suppression of Nitrate Reductase and Nitrite Reductase in Transgenic Tobacco Plants," *Plant Physiol*. 112:1447-1456).

Transgenic line 30-18.9 is a tobacco transgenic line and is post-transcriptionally silenced for both transgene encoded and endogenous nitrate reductase *(Nia)* genes and has been previously described (J.C. Palauqui and H. Vaucheret (1995) "Field Trial Analysis of Nitrate Reductase Co-suppression: A Comparative Study of 38 Combinations of Transgene Loci," *Plant Mol. Biol*. 29:149-159).

### Example 7

Transgenic tobacco plants (*Nicotiana tabacum*) expressing the rgs-CaM sequence and control tobacco plants of that same variety are crossed with transgenic tobacco plants expressing a replicating RNA comprising a portion of the genomic RNA of potato virus X (PVX) with the *uidA* reporter gene inserted in place of the PVS coat protein gene (transgenic line 155; described by Angell (1997) *EMBO J.* 12:3675-3684). The expression of the betaglucuronidase (GUS) encoded by the *uidA* gene in the 155 line is monitored in each of the crosses by histochemical staining as described by Anandalakshmi *et al*. (1998) *Proc. Natl. Acad. Sci. USA* 95:13079-13084. The offspring of the cross between line 155 and the nontransformed control plants display little of the blue staining caused by GUS activity in this assay, indicating that the plants remain silenced in the hemizygous state. In contrast, the offspring of the cross of line 155 and rgs-CaM transformed lines display an intense blue stain after only a short incubation. This result indicates that silencing of PVS-GUS is reversed in the presence of rgs-CaM expression and suggests that the level of expression provided by the combination of the two transgenes is unexpectedly high. GUS activity levels are measured in offspring by a quantitative fluorometric enzyme assay as described by Jefferson (1987) *Plant Molecular Biology Reporter* 5:387-405. GUS activity in offspring of the cross of line 155 and the nontransformed control plants is low. In contrast, GUS activity in the offspring of the cross of line 155 and rgs-CaM transformants is exceptionally high as compared to those in offspring of the control cross in conventional GUS-expressing transgenic lines such as line T19 (described in English *et al*. (1996) *Plant Cell* 8:179-188). GUS activity levels in the line 155/rgs-CaM combination are at least about two orders of magnitude higher than previously observed in transgenic plants.

### Example 8

In this example, the ability of the rgs-CaM sequence to reverse PTGS and produce enhanced expression is tested in another line (line 163; comprising the PVX genome with the *uidA* reporter gene inserted upstream of the PVX coat protein gene and under control of a repeated coat protein subgenomic promoter as described by Angell and Baulcombe (1997)). Line 163 differs from line 155 in several aspects: 1) line 163 contains the coat protein gene and produces coat protein, whereas line 155 has *uidA* inserted in place of the coat protein gene and therefore produces no coat protein; 2) the 25K triple block movement protein coding region of line 163 has a deletion that renders the RNA unable to move cell to cell, whereas this region is wild type in line 155. Since both the triple block proteins and the coat protein of PVX are required for movement of the viral RNA from cell to cell, both RNAs are confined to the cell where they are expressed, but for different reasons; 3) the level of GUS expression in line 163 is significantly lower than that in line 155 as previously shown by Angell and Baulcombe (1997). The lower levels of GUS activity in line 163 may be due to expression of the transgene from a repeated subgenomic promoter, to the production of coat protein, which may serve as a suppressor of viral replication, or to enhanced silencing triggered by the 163 line.

Line 163 is crossed with rgs-CaM transgenic plants and with control nontransformed plants of the same genetic background. The offspring of the cross between line 163 and the nontransformed control plants display little of the blue staining caused by GUS activity in this assay indicating that the plants remained silenced in the hemizygous state. In contrast, the offspring of the cross of line 163 and rgs-CaM transformants display an intense blue stain after only a short incubation, indicating that silencing of PVS-GUS is reversed in the presence of the viral sequence. GUS activity levels are measured as described in Example 1.

### Example 9

In this example, a transgenic line is produced comprising a replicating portion of the PVX genome similar to line 155, but with an endogenous plant gene such as phytoene desaturase in place of the coat protein gene (instead of the reporter gene encoding GUS as in line 155). This PVX-phytoene desaturase plant is crossed with the rgs-CaM transgenic line expressing the rgs-CaM suppressor of silencing and the offspring show reversal of PVX-phytoene desaturase silencing and enhanced expression of the phytoene desaturase gene product as compared to controls.

Other endogenous plant genes can be similarly expressed at enhanced levels by this method.

### Example 10

The present example sets forth an exemplary method for enhancing expression of an endogenous plant gene or a foreign gene (or a portion of a foreign or endogenous gene) that has been introduced to a plant as a fusion to a viral protein expressed from a viral vector. A viral vector expressing a foreign gene or an endogenous plant sequence as a fusion to the coat protein of the virus, such as the vector described in Sugiyama. Hamamoto, Takemoto, Watanabe, Okada (1995) "Systematic Production of Foreign Peptides on the Particle Surface of Tobacco Mosaic Virus," *FEBS Lett*. 359:247-250, is one such example. The viral vector may be used to infect a transgenic plant host that supplies the booster sequence via expression from stably incorporated DNA copies of said booster sequence, for example the rgs-CaM transgenic tobacco plants described herein (Example 2). The expression of the foreign peptides fused to the viral coat protein is enhanced as compared to expression of the foreign peptides in plants not comprising the rgs-CaM booster sequence.

Other gene product-producing vectors to which the presently described booster sequence could be supplied include those described in Hamamoto *et al*. (1993) *Bio*/*Technology* 11:930-932 and Takamatsu *et al.* (1990) *FEBS Lett*. 269:73-76.

### Example 11

The effect of rgs-CaM expression on transgene induced gene silencing is further examined using another transgenic line exhibiting a gene silencing phenotype. In this example, the silenced line (line 106-14) is crossed with rgs-CaM expressing lines or control plants, and plants from the F1 generation are then assayed for expression of the previously silenced gene.

The transgenic line 106-14 was constructed at NC State University and contains over 100 copies of the *uidA* gene under control of the cauliflower mosaic virus promoter and flanked by matrix attachment regions that are reported to interfere with transcriptional gene silencing. These plants express very high levels of GUS activity as seedlings and then silence developmentally, so that older plants fail to express detectable levels of GUS activity and are resistant to PVX-GUS. Developmental silencing is characteristic of post-transcriptional silencing. Line 106-14 is crossed with an rgs-CaM expressing line, and the offspring are susceptible to PVX-GUS and express high levels of GUS activity even in mature flowering plants. In contrast, offspring of control crosses with nontransformed tobacco (cv xanthi) and vector-only transformed tobacco (cv havana 425) fail to produce detectable GUS activity in leaves of mature plants. This result is another example of the ability of the booster sequence to interfere with gene silencing and thereby boost expression of a gene product from a transgene.

### Example 12

In certain systems it is desirable to induce PTGS to prevent expression of endogenous genes and thereby cause a desired phenotype in the plant. However, it is known in the art that silencing is not always complete, and expression of the target sequence desired to be silenced occurs at reduced but yet still undesirable levels. In other cases, gene silencing is reversed, for example, in some instances of viral infection where HC-Pro produced by the viral pathogens has been shown to reverse gene silencing in infected plants. Advantageously, the rgs-CaM booster sequence can be modified using known techniques such as site-directed mutagenesis, synthetic construction of variant sequences, or *Bal*31 exonuclease to cause deletions or mutations of the sequence to yield polynucleotides which encode variant rgs-CaM peptides that retain the ability to interact with HC-Pro, yet fail to mediate the interference with gene silencing. Plants into which such polynucleotides are introduced and expressed have more stable PTGS even in the face of viral infection.

Using the methods of the invention, increased expression of target sequences can be realized. Thus, plants and plant cells and tissues having enhanced production of plant proteins, transgenic proteins, and the like can be produced. Because co-suppression is a general phenomenon in plants, the methods have wide applicability in all plants.

### References

Anandalakshmi, R., Pruss, G.J., Ge, X., Marathe, R., Mallory, A.C., Smith, T.H. and Vance, V.B. (1998) "A viral suppressor of gene silencing in plants," *Proc. Natl. Acad Sci. USA* **95**:13079-13084.
Al-Kaff, N..S., Covey, S.N., Kreike, M.M., Page, A.M., Pinder, R. & Dale, P.J. (1998) "Transcriptional and post transcriptional plant gene silencing in response to a pathogen," *Science* **279**:2113-2115.
Baulcombe, D.C. (1999) "Fast forward genetics based on virus-induced gene silencing," *Gurr. Opin. Plant Biol*. **2**:109-113.
Bingham, P. M. (1997) "Cosuppression comes to the animals," *Cell* **90**:385-387.
Brigneti, G., Voinnet, 0., Li, W.-X., Ji, L.-H., Ding, S.-W. and Baulcombe, D. (1998) "Viral pathogenicity determinants are suppressors of transgene silencing in *Nicotiana benthamiana," EMBO J*. **17**:6739-6746.
Carrington *et al*. (1990) *EMBO. J*. **9**:1347-1353.
Dearolf, C.R., Hersperger, E. and Sheam A. (1988) "Developmental consequences of awdb3, a cell-autonomous lethal mutation of Drosophila induced by hybrid dysgenesis," *Dev. Biol*. **129**:159-168.
de Ia Rosa, A., Williams, R.L. and Steeg, P.S. (1995) "Nm23/nucleoside diphosphate kinase: toward a structural and biochemical understanding of its biological functions," *Bioassays* 17:53-62.
Depicker, A. and Van Montagu, M. (1997) "Post-transcriptional gene silencing in plants," *Gurr. Opin. Cell Biol*. 9:373-382.
Engel, M., Seifert, M., Theisinger, B., Seyfert, U., and Welter, C. (1998) "Glyceraldehyde3-phosphate dehydrogenase and NM23-HI/nucleoside diphosphate kinase A. Two old enzymes combine for the novel Nm23 protein phosphotransferase function," *J. Biol. Chem.* **273**:20058-20065.
Fire, A., Xu, S., Montgomery, M.K., Kostas, S.A., Driver, S.E. and Mello, C.C. (1998) "Potent and specific genetic interference by double stranded RNA in *Caenorhabditis elegens.," Nature* 391:806-811.
Fire, A., *Trends Genet.* 15, 358 (1999).
Grant, S. (1999) "Dissecting the mechanisms of post transcriptional gene silencing: Divide and conquer," *Cell* **96**:303-306.
Howlett, A.R., Peterson, O.W., Steeg, P.S., and Bissell, M.J. (1994) "A novel function for the nm23-H1 gene: overexpression in human breast carcinoma cells leads to the formation of basement membrane and growth arrest," *J. Natl. Cancer Inst*. **86**:1838-1844.
Jones. A.L., Thomas. C.L. and Maule, A.J. (1998) "De novo methylation and cosuppression induced by a cytoplasmically replicating plant RNA virus," *EMBO J.* 17:6385-6393.
Kasschau, K.D. and Camngton, J.C. (1998) "A counterdefensive strategy of plant viruses: Suppression of post transcriptional gene silencing." *Cell* **95**:461-470.
Kennerdell, J.R. and Carthew, R.W. (1998) "Use of dsRNA-mediated genetic interference to demonstrate that frizzled and frizzled 2 act in the wingless pathway," *Cell* **95**:1017-1026.
Kumagai, M.H., Donson, J., Della-Cioppa, G.. Harvey, D., Hanley, K., and Grill, L.K. (1995) "Cytoplasmic inhibition of carotenoid biosynthesis with virus-derived RNA," *Proc. Natl. Acad. Sci USA* **92**:1679-1683.
Matzke, M.A. and Matzke, A.J.M. (1995) "Homology-dependent gene silencing in transgenic plants: what does it really tell us," *Trends in Genetics II, 1-3.*
Metzlaff, M., O'Dell, M., Cluster, P.D.. and Flavell, R.B. (1997) "RNA-mediated RNA degradation and chalcone synthase A silencing in Petunia," *Cell* **88**:845-854.
Montgomery, M. K. and Fire, A. (1998) "Double stranded RNA as a mediator in sequence specific genetic silencing and co-suppression," *Trends Genet.* 14:255-258.
Palauqui, J.C. and H. Vaucheret (1995) "Field Trial Analysis of Nitrate Reductase Co-suppression: A Comparative Study of 38 Combinations of Transgene Loci," *Plant Mol. Biol*. 29:149-159.
Palauqui, J.C., T. Elmayan, F. Dorlhac de Borne, P. Crete, C. Charles and H. Vaucheret (1996) "Frequencies, Timing, and Spatial Patterns of Co-suppression of Nitrate Reductase and Nitrite Reductase in Transgenic Tobacco Plants," *Plant Physiol.* 112:1447-1456.
Postel, E. H., Berbench, S.J., Flint, S.J., and Ferrone, C.A. (1993) "Human c-myc transcription factor PuF identified as nm-23-H2 nucleoside diphosphate kinase, a candidate suppressor of tumor metastasis," *Science* 261:478-480.
Pruss, G., Ge, X., Shi, X.M., Camngton, J.C., and Vance, V.B. (1997) "Plant viral synergism: the potyviral genome encodes a broad-range pathogenicity enhancer that transactivates replication of heterologous viruses," *Plant Cell* **9**:1-11.
Ratcliff, F., Harrison, B., and Baulcombe, D. (1997) "A similarity between viral defense and gene silencing in plants," *Science* **276**:1558-1560.
Romano. N. and Macmo, C. (1992) "Quelling: transient inactivation of gene expression in *Neurospora* crassa by transformation with homologous sequences," *Mol. Microbiol.* 6:3343-3353.
Rudd and Franklin-Tong, 1999.
Ruiz, M.T., Voinnet, 0., and Baulcombe, D.C. (1998) "Initiation and amintenance of virus-induced gene silencing," *Plant Cell* **10**:937-946.
Rumz, F., Vayssie, L., Klotz, C., Sperling, L., and Madeddu, L. (1998) "Homology-dependent gene silencing in Paramecium," *Mol. Biol. Cell* **9**:931-943.
Sharp, P.A., *Genes Dev*., 13, 139 (1999).
Sharp, P.A. and P.D. Zamore, *Science* 287,2431 (2000).
Snedden and Hillel, 1999.
Vance, V.B. (1991) "Replication of potato virus X RNA is altered in coinfections with potato virus Y," *Virology* **182**:486-494.
Vance, V.B., Berger, P.H., Canington, J.C., Hunt, A.G., and Shi, X.M. (1995) "5' proximal potyviral sequences mediate potato virus X/potyviral synergistic disease in transgenic tobacco," *Virology* **206**:583-590.
Waterhouse, P.M., Graham, MW. and Wang, M. (1998) Virus resistance and gene silencing in plants can be induced by simultaneous expression of sense and antisense RNA," *Proc. Natl. Acad Sci. USA* **95**:13959-13964.
Wianny, F. and M. Zemicka-Goetz, *Nature Cell. Biol*. 2, 70 (2000).

## Claims

1. A method of expressing a foreign gene or an endogenous plant gene that has been introduced into plant cells, plant protoplasts or whole plants, the method comprising supplying to said plant cells, plant protoplasts or whole plants a booster sequence comprising a polynucleotide encoding a rgs-CaM polypeptide that has the ability to act as a suppressor of post-transcriptional gene silencing, wherein the polynucleotide comprises:
(a) a nucleotide sequence that encodes at least a fragment of the polypeptide of SEQ ID NO:2; or
(b) a nucleotide sequence having at least 70% identity to SEQ ID NO: 1.

2. The method of claim 1, wherein the introduced gene is a foreign gene that was not naturally occurring in the plant cells, plant protoplasts or whole plants.

3. The method of claim 1, wherein the introduced gene is an endogenous plant gene that was naturally occurring in the plant cells, plant protoplasts or whole plants prior to being introduced as one or more additional copies of the endogenous gene.

4. The method of claim 1, wherein the booster sequence is expressed independently.

5. The method of claim 1, wherein the booster sequence is fused to other sequences.

6. The method of claim 1, wherein the introduced gene is introduced via a viral expression vector and the booster sequence is supplied by expression from the same viral vector.

7. The method of claim 1, wherein the introduced gene is introduced via a viral expression vector and the booster sequence is supplied by expression of one or more DNA copies of the booster sequence stably incorporated into the plant's genome.

8. The method of claim 1, wherein the booster sequence is expressed from a transient expression system.

9. The method of claim 1, which comprises using a two-component viral vector system of which one viral component expresses the booster sequence and the other viral component expresses the introduced gene.

10. The method of claim 1, wherein the introduced gene is introduced into the plant genome via any mode of stable transformation of one or more copies of the introduced gene, and the booster sequence is supplied prior to introduction of the introduced gene via stable transformation procedures.

11. The method of claim 1, wherein the introduced gene is introduced into the plant genome via any mode of stable transformation of one or more copies of the introduced gene into the plant genome, and the booster sequence is supplied during the process of introduction of the introduced gene via stable transformation procedures.

12. The method of claim 1, wherein the introduced gene is introduced into the plant genome via any mode of stable transformation of one or more copies of the introduced gene into the plant genome, and the booster sequence is supplied after introduction of the introduced gene via stable transformation procedures.

13. The method of any preceding claim, wherein the booster sequence comprises a nucleotide sequence that encodes SEQ ID NO:2.

14. The method of claim 13, wherein the booster sequence comprises SEQ ID NO:1.

15. An isolated polynucleotide encoding a rgs-CaM polypeptide that has the ability to act as a suppressor of post-transcriptional gene silencing, wherein said polynucleotide comprises:
(a) a nucleotide sequence that encodes at least a fragment of the polypeptide of SEQ ID NO:2; or
(b) a nucleotide sequence having at least 70% identity to SEQ ID NO: 1.

16. A polynucleotide according to claim 15, which encodes SEQ ID NO: 2.

17. A polynucleotide according to claim 15, which comprises SEQ ID NO: 1.

18. A rgs-CaM polypeptide that has the ability to act as a suppressor of post-transcriptional gene silencing, wherein the polynucleotide comprises:
(a) at least a fragment of the polypeptide of SEQ ID NO:2; or
(b) an amino acid sequence encoded by a nucleotide sequence having at least 70% identity to SEQ ID NO:1.

19. A polypeptide according to claim 18, comprising SEQ ID NO: 2.

20. A transformed plant produced by the method of any of claims 1 to 14, and which comprises the booster sequence.

21. A transformed seed of the plant of claim 20, and which comprises the booster sequence.

22. A transformed plant cell produced by the method of any of claims 1 to 14, and which comprises the booster sequence.

23. A transformed plant comprising, stably incorporated in its genome, a polynucleotide according to any of claims 15 to 17.

24. A transformed seed of the plant of claim 23, which comprises said polynucleotide stably incorporated in its genome.

25. A transformed plant cell comprising, stably incorporated in its genome, a polynucleotide according to any of claims 15 to 17.

## Patentansprüche

1. Verfahren zum Exprimieren eines fremden Gens oder eines endogenen Pflanzengens, das in Pflanzenzellen, Pflanzenprotoplasten oder vollständige Pflanzen eingeführt worden ist, wobei das Verfahren das Versorgen der Pflanzenzellen, Pflanzenprotoplasten oder vollständigen Pflanzen mit einer Boostersequenz umfasst, die ein Polynucleotid umfasst, das für ein rgs-CaM-Polypeptid kodiert, das die Fähigkeit aufweist, als Suppressor einer posttranskriptionalen Gen-Abschaltung zu wirken, wobei das Polynucleotid folgendes umfasst:
(a) eine Nucleotidsequenz, die zumindest für ein Fragment des Polypeptids von SEQ ID NO: 2 kodiert; oder
(b) eine Nucleotidsequenz mit einer mindestens 70%igen Identität zu SEQ ID NO: 1.

2. Verfahren nach Anspruch 1, wobei es sich bei dem eingeführten Gen um ein fremdes Gen handelt, das nicht natürlicherweise in den Pflanzenzellen, Pflanzenprotoplasten oder vollständigen Pflanzen auftritt.

3. Verfahren nach Anspruch 1, wobei es sich bei dem eingeführten Gen um ein endogenes Pflanzengen handelt, das natürlicherweise in den Pflanzenzellen, Pflanzenprotoplasten oder vollständigen Pflanzen vorkommt, bevor eine oder mehrere zusätzliche Kopien des endogenen Gens eingeführt werden.

4. Verfahren nach Anspruch 1, wobei die Boostersequenz in unabhängiger Weise exprimiert wird.

5. Verfahren nach Anspruch 1, wobei die Boostersequenz mit anderen Sequenzen fusioniert ist.

6. Verfahren nach Anspruch 1, wobei das eingeführte Gen über einen viralen Expressionsvektor eingeführt wird und die Boostersequenz durch Expression aus dem gleichen viralen Vektor zur Verfügung gestellt wird.

7. Verfahren nach Anspruch 1, wobei das eingeführte Gen über einen viralen Expressionsvektor eingeführt wird und die Boostersequenz durch Expression von einer oder mehreren DNA-Kopien der Boostersequenz, die in stabiler Weise in das Pflanzengenom eingebaut ist, zur Verfügung gestellt wird.

8. Verfahren nach Anspruch 1, wobei die Boostersequenz aus einem vorübergehenden Expressionssystem exprimiert wird.

9. Verfahren nach Anspruch 1, welches die Verwendung eines viralen Zweikomponenten-Vektorsystems umfasst, von dem eine virale Komponente die Boostersequenz exprimiert und die andere virale Komponente das eingeführte Gen exprimiert.

10. Verfahren nach Anspruch 1, wobei das eingeführte Gen in das Pflanzengenom über einen beliebigen Modus der stabilen Transformation von einer oder mehreren Kopien des eingeführten Gens eingeführt wird und die Boostersequenz vor der Einführung des eingeführten Gens über stabile Transformationsvorgänge zur Verfügung gestellt wird.

11. Verfahren nach Anspruch 1, wobei das eingeführte Gen in das Pflanzengenom über einen beliebigen Modus der stabilen Transformation von einer oder mehreren Kopien des eingeführten Gens in das Pflanzengenom eingeführt wird und die Boostersequenz während des Verfahrens der Einführung des eingeführten Gens über stabile Transformationsvorgänge zur Verfügung gestellt wird.

12. Verfahren nach Anspruch 1, wobei das eingeführte Gen in das Pflanzengenom über einen beliebigen Modus der stabilen Transformation von einer oder mehreren Kopien des eingeführten Gens in das Pflanzengenom eingeführt wird und die Boostersequenz nach Einführung des eingeführten Gens über stabile Transformationsvorgänge zur Verfügung gestellt wird.

13. Verfahren nach einem der vorstehenden Ansprüche, wobei die Boostersequenz eine Nucleotidsequenz umfasst, die für SEQ ID NO: 2 kodiert.

14. Verfahren nach Anspruch 13, wobei die Boostersequenz SEQ ID NO: 1 umfasst.

15. Isoliertes Polynucleotid, kodierend für ein rgs-CaM-Polypeptid, das die Fähigkeit besitzt, als Suppressor einer posttranskriptionalen Genabschaltung zu wirken, wobei das Polynucleotid folgendes umfasst:
(a) eine Nucleotidsequenz, die zumindest für ein Fragment des Polypeptids von SEQ ID NO: 2 kodiert; oder
(b) eine Nucleotidsequenz mit einer mindestens 70%igen Identität zu SEQ ID NO: 1.

16. Polynucleotid nach Anspruch 15, das für SEQ ID NO: 2 kodiert.

17. Polynucleotid nach Anspruch 15, das SEQ ID NO: 1 umfasst.

18. rgs-CaM-Polypeptid, das die Fähigkeit besitzt, als Suppressor der posttranskriptionalen Genabschaltung zu wirken, wobei das Polynucleotid folgendes umfasst:
(a) mindestens ein Fragment des Polypeptids von SEQ ID NO: 2; oder
(b) eine Aminosäuresequenz, die durch eine Nucleotidsequenz mit einer Identität von mindestens 70 % zu SEQ ID NO: 1 kodiert wird.

19. Polypeptid nach Anspruch 18, umfassend SEQ ID NO: 2.

20. Transformierte Pflanze, die nach dem Verfahren von einem der Ansprüche 1 bis 14 hergestellt worden ist und die die Boostersequenz umfasst.

21. Transformierter Samen der Pflanze nach Anspruch 20, der die Boostersequenz umfasst.

22. Transformierte Pflanzenzelle, die nach dem Verfahren von einem der Ansprüche 1 bis 14 hergestellt worden ist und die Boostersequenz umfasst.

23. Transformierte Pflanze, umfassend ein Polynucleotid nach einem der Ansprüche 15 bis 17, das in stabiler Weise in das Genom der Pflanze eingebaut ist.

24. Transformierter Samen der Pflanze nach Anspruch 23, der das Polynucleotid umfasst, das in stabiler Weise in sein Genom eingebaut ist.

25. Transformierte Pflanzenzelle, die ein Polynucleotid nach einem der Ansprüche 15 bis 17 umfasst, das in stabiler Weise in ihr Genom eingebaut ist.

## Revendications

1. Procédé d'expression d'un gène étranger ou d'un gène endogène de végétal qui a été introduit dans des cellules végétales, des protoplastes végétaux ou des plantes entières, le procédé comportant l'étape qui consiste à délivrer auxdites cellules végétales, protoplastes végétaux ou plantes entières une séquence de rappel (séquence « booster ») qui comprend un polynucléotide qui code pour un polypeptide rgs-CaM qui a la capacité d'agir comme suppresseur de l'inhibition du gène après sa transcription, dans lequel le polynucléotide comprend :
(a) une séquence de nucléotides qui code pour au moins un fragment du polypeptide SEQ ID NO:2 ou
(b) une séquence de nucléotides qui présente au moins 70 % d'identité avec la SEQ ID NO:1.

2. Procédé selon la revendication 1, dans lequel le gène introduit est un gène étranger qui n'existe pas naturellement dans les cellules végétales, les protoplastes végétaux ou les plantes entières.

3. Procédé selon la revendication 1, dans lequel le gène introduit est un gène végétal endogène qui est naturellement présent dans les cellules végétales, les protoplastes végétaux ou les plantes entières avant d'être introduit sous la forme d'une ou de plusieurs copies supplémentaires du gène endogène.

4. Procédé selon la revendication 1, dans lequel la séquence de rappel est exprimée indépendamment.

5. Procédé selon la revendication 1, dans lequel la séquence de rappel est fusionnée à d'autres séquences.

6. Procédé selon la revendication 1, dans lequel le gène introduit est introduit par un vecteur d'expression virale, la séquence de rappel étant délivrée par expression depuis le même vecteur viral.

7. Procédé selon la revendication 1, dans lequel le gène introduit est introduit par l'intermédiaire d'un vecteur d'expression virale, la séquence de rappel étant délivrée par expression d'une ou de plusieurs copies de l'ADN de la séquence de rappel incorporées de manière stable dans le génome de la plante.

8. Procédé selon la revendication 1, dans lequel la séquence de rappel est exprimée par un système d'expression transitoire.

9. Procédé selon la revendication 1 qui comprend l'utilisation d'un système de vecteur viral à deux composants dans lequel un composant viral exprime la séquence de rappel et l'autre composant viral exprime le gène introduit.

10. Procédé selon la revendication 1, dans lequel le gène introduit est introduit dans le génome de la plante par un mode quelconque de transformation stable d'une ou de plusieurs copies du gène introduit, la séquence de rappel étant fournie par des procédures de transformation stable avant l'introduction du gène introduit.

11. Procédé selon la revendication 1, dans lequel le gène introduit est introduit dans le génome de la plante par un mode quelconque de transformation stable d'une ou de plusieurs copies du gène introduit dans le génome de la plante, la séquence de rappel étant fournie par des procédures de transformation stable pendant l'opération d'introduction du gène introduit.

12. Procédé selon la revendication 1, dans lequel le gène introduit est introduit dans le génome de la plante par un mode quelconque de transformation stable d'une ou de plusieurs copies du gène introduit dans le génome de la plante, la séquence de rappel étant délivrée après l'opération d'introduction du gène introduit, par des procédures de transformation stables.

13. Procédé selon l'une quelconque des revendications précédentes, dans lequel la séquence de rappel comprend une séquence de nucléotides qui code pour la SEQ ID NO:2.

14. Procédé selon la revendication 13, dans lequel la séquence de rappel comprend la SEQ ID NO:1.

15. Polynucléotide isolé qui code pour un polypeptide rgs-CaM qui a la capacité d'agir comme suppresseur de l'inhibition d'un gène après sa transcription, ledit polynucléotide comprenant :
(a) une séquence de nucléotides qui code pour au moins un fragment du polypeptide de SEQ ID NO:2 ou
(b) une séquence de nucléotides qui a au moins 70 % d'identité par rapport à la SEQ ID NO:1.

16. Polynucléotide selon la revendication 15, qui code pour la SEQ ID NO:2.

17. Polynucléotide selon la revendication 15, qui comprend la SEQ ID NO:1.

18. Polypeptide rgs-CaM qui a la capacité d'agir comme suppresseur de l'inhibition d'un gène après sa transcription, le polynucléotide comprenant :
(a) au moins un fragment du polypeptide de SEQ ID 2 ou
(b) une séquence d'acides aminés codée par une séquence de nucléotides qui présente au moins 70 % d'identité par rapport à la SEQ ID NO:1.

19. Polypeptide selon la revendication 18, qui comprend la SEQ ID NO:2.

20. Plante transformée produite par le procédé selon l'une quelconque des revendications 1 à 14, qui comprend la séquence de rappel.

21. Semence transformée de la plante selon la revendication 20, qui comprend la séquence de rappel.

22. Cellule transformée de plante produite par le procédé selon l'une quelconque des revendications 1 à 14, qui comprend la séquence de rappel.

23. Plante transformée qui comprend, incorporé de manière stable dans son génome, un polynucléotide selon l'une quelconque des revendications 15 à 17.

24. Semence transformée de plante selon la revendication 23, qui comprend ledit polynucléotide incorporé de manière stable dans son génome.

25. Cellule transformée de plante, qui comprend, incorporé de manière stable dans son génome, un polynucléotide selon l'une quelconque des revendications 15 à 17.
